(19) Europäisches Patentamt
European Patent Office
Office européen des brevets

(11) **EP 4 564 007 A1**

(12) **EUROPEAN PATENT APPLICATION**
published in accordance with Art. 153(4) EPC

(43) Date of publication:
**04.06.2025  Bulletin 2025/23**

(21) Application number: **23841489.0**

(22) Date of filing: **15.06.2023**

(51) International Patent Classification (IPC):
***G01N 33/86*** (2006.01)  ***G01N 33/48*** (2006.01)
***G16H 10/40*** (2018.01)

(52) Cooperative Patent Classification (CPC):
**G01N 33/48; G01N 33/86; G16H 10/40**

(86) International application number:
**PCT/JP2023/022245**

(87) International publication number:
**WO 2024/018789 (25.01.2024 Gazette 2024/04)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **21.07.2022  JP 2022116536
25.01.2023  JP 2023009620**

(71) Applicant: **HITACHI HIGH-TECH CORPORATION
Tokyo 105-6409 (JP)**

(72) Inventors:
• **MINEMURA Hiroyuki
Tokyo 100-8280 (JP)**
• **YAMAMOTO Kyoko
Tokyo 100-8280 (JP)**
• **YABUTANI Chie
Tokyo 105-6409 (JP)**
• **UMEDA Mariko
Tokyo 100-8280 (JP)**

(74) Representative: **MERH-IP Matias Erny Reichl
Hoffmann
Patentanwälte PartG mbB
Paul-Heyse-Straße 29
80336 München (DE)**

(54) **METHOD FOR ESTIMATING CAUSE OF PROLONGATION OF BLOOD COAGULATION TIME, AND INFORMATION PROCESSING DEVICE**

(57)    A cause of prolongation of a blood clotting time for a test sample is estimated. A method for estimating a cause of prolongation of a blood clotting time includes: acquiring a clot waveform showing a change in light amount over time according to a coagulation reaction of a reaction liquid obtained by mixing a test sample and a reagent (S301b); acquiring a first waveform by performing before-after differentiation processing on the clot waveform (S302b) ; acquiring first and second fitted waveforms by performing fitting processing on the clot waveform and the first waveform (S303b, S304b) ; acquiring a third waveform by performing before-after differentiation processing on the second fitted waveform (S305b); acquiring first to third normalized waveforms by normalizing the first and second fitted waveforms and the third waveform, respectively, on a light amount axis and a time axis (S306b); and extracting a feature from each of the first to third normalized waveforms (S307b) ; and estimating a cause of prolongation of a blood clotting time for the test sample based on a known feature and the extracted feature.

*FIG. 3B*

MEASURE COAGULATION REACTION

| READ CLOT WAVEFORM (WaveSrc0) DATA | S301b |

| GENERATE WaveSrc1 | S302b |

| FIT WaveSrc0 (GENERATE Wave0) | S303b |

| FIT WaveSrc1 (GENERATE Wave1) | S304b |

| GENERATE Wave2 | S305b |

| PERFORM NORMALIZATION PROCESSING ON Wave0, Wave1, and Wave2 BASED ON NRC METHOD (ON BOTH TIME AXIS AND LIGHT AMOUNT AXIS) (GENERATE WaveNor0, WaveNor1 AND WaveNor2) | S306b |

| EXTRACT FEATURE | S307b |

ESTIMATE IDENTIFICATION

EP 4 564 007 A1

**Description**

Technical Field

The present disclosure relates to a method for estimating a cause of prolongation of a blood clotting time and an information processing device.

Background Art

Blood coagulation tests are performed for the purpose of grasping the pathology of the blood coagulation and fibrinolysis system, diagnosing disseminated intravascular coagulation (DIC), confirming the effectiveness of thrombosis treatment, diagnosing hemophilia, and the like. In particular, blood clotting time measurement is to measure a time (hereinafter, referred to as a blood clotting time) until a fibrin clot is formed by mixing a sample and a reagent. The blood clotting time is prolonged if there is a congenital or acquired abnormality in blood coagulation ability. Examples of the cause of prolongation of the blood clotting time include a deficiency of a blood coagulation factor (deficient type), an inhibition of a blood coagulation reaction by an antibody against a blood coagulation factor, a component (e.g., phospholipid) in a reagent for measuring a blood clotting time (inhibitor type), or the like. Since the treatment method differs depending on the cause of prolongation of the blood clotting time, it is necessary to identify the cause of prolongation. However, it is not possible to identify the cause of prolongation only by measuring the blood clotting time.

A representative method for identifying a cause of prolongation of a blood clotting time is a cross mixing test. In the cross mixing test, blood clotting times of a plurality of plasma samples obtained by mixing a test sample and a normal sample at different ratios (e.g. 10:0, 9:1, 8:2, 5:5, 2:8, 1:9, 0:10) are measured. Measurements are performed immediately after the sample is prepared (immediate-type) and after the sample is heated (incubated) for 2 hours (delayed-type). The obtained blood clotting times are plotted on the vertical axis and the test sample mixing ratios are plotted on the horizontal axis, and the plots are connected by a line to form a graph. The cause of prolongation is determined from the shapes of the graphs of immediate-type and delayed-type measurements. This determination is qualitative and requires a person who makes the determination to have a high level of experience. In addition, the complicated sample preparation and incubation procedures at the time of examination involved in the cross mixing test place a burden on an examiner. Furthermore, additional blood collection is required for this examination, which places a burden on a patient.

As an evaluation method for reducing the burden on the examiner and the burden on the patient and providing a more quantitative determination result, it has been proposed to identify a cause of prolongation by analyzing a clot waveform. The target clot waveform is a waveform acquired by measuring light, in which a change in turbidity over time is recorded as a fibrin clot is formed.

PTL 1 discloses a method in which a cause of prolongation of a blood clotting time in a test sample is estimated by discriminating whether the cause of prolongation is of a deficient type or an inhibitor type, using a feature extracted from a clot waveform acquired from a plasma sample obtained by mixing a test sample and a normal sample and a derivative waveform thereof. PTL 2 discloses a method in which it is determined whether there is a blood coagulation abnormality in a test sample an activity value (concentration) of a blood coagulation factor is estimated by template matching using 50 parameters related to the center of gravity of a waveform obtained from a first derivative of a clot waveform acquired from the test sample. In particular, a method is disclosed in which a concentration of a blood coagulation factor VIII (FVIII) and a concentration of a blood coagulation factor IX (FIX) are estimated, and which blood coagulation factor is deficient in a test sample is identified and estimated. Qualitative and quantitative abnormalities of FVIII are called hemophilia A. Qualitative and quantitative abnormalities of FIX are called hemophilia B. Which blood coagulation factor is deficient is important because it is related to a selection of a treatment method. PTL 3 discloses an APTT prolongation factor estimation system equipped with a template matching algorithm. NPL 1 discloses a method whether a test sample is of a deficient type, an inhibitor type, or an anticoagulant-added type is identified and estimated by a flowchart using a slope or an area of a part of a waveform obtained from a first derivative of a clot waveform acquired from the test sample, a time width ratio of the waveform, etc. In particular, in a case where the test sample is of the inhibitor type, it is identified and estimated whether the test sample is a lupus anticoagulant (LA)-positive group or a group that expresses an inhibitor to FVIII. LA is one of the inhibitors to phospholipids, and its expression leads to anti-phospholipid antibody syndrome. PTL. 4 describes that a plurality of parameters are extracted from a first derivative waveform and a second derivative waveform of a clot waveform, an estimated concentration of each blood coagulation factor is obtained by multi-variate correlation from the plurality of parameters, and a trained neural network is used for the estimation.

Citation List

Patent Literature

PTL 1: JP 6994528 B2
PTL 2: WO 2020/158948 A1
PTL 3: JP 6811975 B2
PTL 4: US 6524861 B1

Non Patent Literature

NPL 1: D. Shimomura et al., The First-Derivative Curve of the Coagulation Waveform Reveals the Cause of aPTT Prolongation, Clinical and Applied Thrombosis/Hemostasis, Volume 26 (2020) P1-8

Summary of Invention

Technical Problem

(1) An object of the present invention is to identify and estimate which one of a FVIII-deficient sample group, a FIX-deficient sample group, and an LA-positive sample group, which are important for diagnosis of hemophilia A, hemophilia B, and anti-phospholipid antibody syndrome, is a cause of prolongation of a blood clotting time of a test sample, by using a feature extracted by analyzing a clot waveform acquired by measuring a coagulation reaction in a reaction liquid generated by mixing the test sample and a reagent.
(2) The blood clotting time of a sample from a hemophilia patient becomes longer as the severity of the condition increases. It is common knowledge to those skilled in the art that, in order to improve throughput, a device for measuring a blood clotting time terminates a measurement by appropriately determining that a blood coagulation reaction has ended for each sample, and moves onto a measurement for a next sample. As a result, the data length of the clot waveform is different for each sample.

PTL 4 describes that an estimated concentration of each blood coagulation factor is obtained from a plurality of parameters by multi-variate backward correlation and a neural network is used to estimate a case. A clustering technique including the neural network uses a feature of a fixed data length as input data. Currently known clustering methods, such as a neural network, a mixed Gaussian distribution, and a K-means method, are techniques that assume that the number of pieces of input data is fixed. When any of the typical clustering techniques including them are used for analyzing a clot waveform, the clustering technique functions only when the clot waveform has a fixed data length by performing appropriate preprocessing on the clot waveform data having a variable data length. As an example of preprocessing for such a variable data length, data compression technology, zero padding technology, and the like are widely known as excellent preprocessing for image and voice recognition. If it is possible to estimate a cause of prolongation of a blood clotting time through a neural network directly using a clot waveform of only a test sample or a series of clot waveform groups obtained by performing a cross mixing test using a plurality of mixed samples obtained by mixing the test sample and a normal sample at a predetermined ratio, it is possible to estimate a cause of prolongation of a blood clotting time based on not only an experimentally determined feature but also hidden information included in the clot waveform. In order to achieve this, it is necessary to perform preprocessing suitable for discriminating a clot waveform having a variable data length by a neural network, demonstrate that practical identification accuracy is obtained by the neural network using the clot waveform or the clot waveform group, and disclose a configuration of the neural network and specific numerical values such as hyper parameters for achieving the same.

Therefore, an object of the present disclosure is to estimate a cause of prolongation of a blood clotting time for a test sample using a feature extracted from a clot waveform showing a change in light amount over time according to a coagulation reaction of a reaction liquid generated by mixing the test sample and a reagent.

Another object of the present disclosure is to estimate a cause of prolongation of a blood clotting time with practical accuracy using a neural network by performing suitable preprocessing on a clot waveform having a variable data length.

Solution to Problem

A method for estimating a cause of prolongation of a blood clotting time according to the present disclosure includes: acquiring a clot waveform showing a change in light amount over time according to a coagulation reaction of a reaction liquid generated by mixing a test sample composed of plasma separated from blood acquired from a subject and a reagent; acquiring a first waveform by performing before-after differentiation processing on the clot waveform; acquiring a first fitted waveform by performing fitting processing on the clot waveform; acquiring a second fitted waveform by performing fitting processing on the first waveform; acquiring a third waveform by performing before-after differentiation processing on the second fitted waveform; acquiring a first normalized waveform, a second normalized waveform, and a third normalized waveform by normalizing the first fitted waveform, the second fitted waveform, and the third waveform, respectively, on a light amount axis and a time axis; extracting a feature from each of the first normalized waveform, the second normalized waveform, and the third normalized waveform; and estimating a cause of prolongation of a blood clotting time for the test sample based on a known feature extracted from a sample group for which a cause of prolongation of a blood clotting time is known and the feature extracted from each of the first normalized waveform, the second normalized waveform, and the third normalized waveform.

A method for estimating a cause of prolongation of a blood clotting time according to the present disclosure is a method for estimating a cause of prolongation of a blood clotting time for a test sample from a clot waveform showing a change in light amount over time according to a coagulation reaction of a reaction liquid generated by mixing the test sample composed of plasma separated from blood acquired from a subject and a reagent, the method including: aligning data lengths of clot waveform data; estimating a plurality of causes of prolongation of blood clotting times based on the clot waveform data, of which the data lengths are aligned, by using a neural network; and presenting a cause of prolongation of a blood clotting time for the test sample based on probabilities belonging to the estimated causes of prolongation of the blood clotting times.

Advantageous Effects of Invention

According to the present disclosure, it is possible to estimate a cause of prolongation of a blood clotting time for a test sample using a feature extracted from a clot waveform showing a change in light amount over time according to a coagulation reaction of a reaction liquid generated by mixing the test sample and a reagent.

Furthermore, according to the present disclosure, by performing suitable preprocessing on a clot waveform having a variable data length, it is possible to estimate a cause of prolongation of a blood clotting time with practical accuracy using the neural network.

Other problems, configurations, and effects that are not described above will be apparent from the following description of embodiments.

Brief Description of Drawings

FIG. 1 is a diagram illustrating clot waveforms of two FVIII-deficient samples and waveforms corresponding to their first derivatives in a first embodiment.

FIG. 2 is a diagram illustrating waveforms obtained by normalizing clot waveforms of a FVIII-deficient sample group, a FIX-deficient sample group, and an LA-positive sample group, waveforms corresponding to first derivatives of the clot waveforms, and waveforms corresponding to second derivatives of the clot waveforms in the first embodiment.

FIG. 3A is a flowchart illustrating a method for estimating a cause of prolongation of a blood clotting time in a comparative example.

FIG. 3B is a flowchart illustrating a method for estimating a cause of prolongation of a blood clotting time in the first embodiment.

FIG. 4 is a diagram illustrating an example of an overall configuration of the automatic analyzer in the first embodiment.

FIG. 5 is a diagram illustrating WaveNor0, WaveNor1, and WaveNor2, which are waveforms obtained by normalizing Wave0, Wave1, and Wave2, and feature vectors, in the first embodiment.

FIG. 6 is a diagram illustrating two-dimensional maps including features of WaveNor0, WaveNor1, and WaveNor2 in the first embodiment.

FIG. 7 is a diagram illustrating an example in which a feature of a test sample is plotted on a two-dimensional map created from features of samples for which causes of prolongation of blood clotting times are known in the first embodiment.

FIG. 8 is a diagram illustrating an example of normalized Wave0, Wave1, and Wave2 of a test sample, an indicator showing probabilities that the test sample belongs to the FVIII-deficient sample group, the FIX-deficient sample group, the LA-positive sample group, the normal sample group, and the unknown sample group, and an identification estimation result.

FIG. 9 is a diagram illustrating a relationship between an analysis unit, a PC, and a communication interface and an example in which a result of a test sample is displayed on a display unit in the first embodiment.

FIG. 10 is a diagram illustrating examples of WaveSrc1 and Wave1 in the first embodiment.

FIG. 11A is a diagram illustrating a preprocessing method for a neural network in a second embodiment.

FIG. 11B is a diagram illustrating a preprocessing method for a neural network in the second embodiment.

FIG. 11C is a diagram illustrating a preprocessing method for a neural network in the second embodiment.

FIG. 12 is a diagram illustrating a structure of a classifier based on a neural network in the second embodiment.

FIG. 13A is an experimental result showing a relationship between a preprocessing method, a dropout of a classifier based on a neural network, and an identification accuracy in the second embodiment.

FIG. 13B is an experimental result showing a relationship between a preprocessing method, a dropout of a classifier based on a neural network, and an identification accuracy in the second embodiment.

FIG. 13C is an experimental result showing a relationship between a preprocessing method, a dropout of a classifier based on a neural network, and an identification accuracy in the second embodiment.

FIG. 14A is an experimental result showing a relationship between a preprocessing method, a hyper parameter of a classifier based on a neural network, and an identification accuracy in the second embodiment.

FIG. 14B is an experimental result showing a relationship between a preprocessing method, a hyper parameter of a classifier based on a neural network, and an identification accuracy in the second embodiment.

FIG. 15 is an experimental result showing that the preprocessing method and the classifier based on the neural network suppress over-learning in the second embodiment.

FIG. 16 is an experimental result showing a difference between a FVIII-deficient sample and a FIX-deficient sample observed from clot waveforms in a fourth embodiment.

FIG. 17A is an experimental result showing a relationship between a maximum value of a first derivative, which is a known index, and a log clotting time.

FIG. 17B is an experimental result showing a relationship between vecl_y and a log clotting time in the fourth embodiment.

FIG. 18A is an experimental result showing a representative waveform of each sample group for WaveNor0 obtained by normalizing a clot waveform of a commercially available sample based on an NRC method in the fourth embodiment.

FIG. 18B is an experimental result showing a representative waveform of each sample group for WaveNor1 obtained by normalizing a clot waveform of a commercially available sample based on the NRC method in the fourth embodiment.

FIG. 18C is an experimental result showing a representative waveform of each sample group for WaveNor2 obtained by normalizing a clot waveform of a commercially available sample based on the NRC method in the fourth embodiment.

FIG. 19 is an experimental result showing a frequency spectrum of WaveNor2 in the fourth embodiment.

FIG. 20 summarizes an example of a feature set in the fourth embodiment.

FIG. 21 is an experimental result in which a commercially available sample group is displayed on feature space #6 in the fourth embodiment.

FIG. 22A is an example of a display showing a summary in which commercially available sample groups are plotted on a feature space in the fourth embodiment.

FIG. 22B is an example of a display showing a discrimination result of one FVIII-deficient sample in the fourth embodiment.

FIG. 23 is a schematic diagram illustrating a method of dimensionally compressing a series of clot waveform data obtained for one test sample into two area indexes $F_1$ and $F_2$ in a cross mixing test in a fifth embodiment.

FIG. 24 is a diagram illustrating a discrimination result of samples in a cross mixing test using a multidimensional mixed Gaussian distribution in the fifth embodiment.

FIG. 25A is a diagram illustrating cluster regions of indexes $(F_1, F_2)$ in the fifth embodiment.

FIG. 25B is a diagram illustrating an experimental result obtained by adding data points to the cluster regions of the indexes $(F_1, F_2)$ in the fifth embodiment.

FIG. 26 is a diagram for explaining indexes $(G_1, G_2)$ in the fifth embodiment.

FIG. 27A is a diagram illustrating an experimental result in which immediate measurement indexes $(F_1, G_1)$ and delayed measurement indexes $(F_2, G_2)$ are plotted, $(F_1, G_1)$ and $(F_2, G_2)$ being obtained by combining an index F calculated from a series of clot waveforms obtained from one sample and an index G calculated from feature space #1 using the same one sample in the fifth embodiment.

FIG. 27B is a diagram illustrating an experimental result in which indexes $(F_1, G_1)$ and $(F_2, G_2)$ obtained from 34 test samples are plotted in the fifth embodiment.

FIG. 28A is an experimental result showing an average shape of orbit data for each cause of prolongation of a blood clotting time obtained by a cross mixing test in the fifth embodiment.

FIG. 28B is a diagram illustrating an experiment result obtained by performing discrimination by separating orbit data of each sample in a cross mixing test into 2 dimensions x 14 spaces in the fifth embodiment.

FIG. 29 is a diagram in which conditions for investigating the total number of samples required for the discrimination method are summarized in the fifth embodiment.

FIG. 30 is a schematic diagram illustrating a relationship between the number of clot waveforms used for discrimination and a worst probability (a minimum value of Pti among all the samples, assuming that Pti is a probability that an i-th sample belongs to the correct sample group cluster) in the fifth embodiment.

FIG. 31 is an example of a display illustrating a discrimination result of a cross mixing test in the fifth embodiment.

Description of Embodiments

Hereinafter, the present embodiment will be described with reference to the drawings. In the drawings, elements that are functionally the same may be denoted by the same numeral. Note that, although the drawings illustrate embodiment and implementation examples in accordance with the principles of the present disclosure, they are for the purpose of understanding the present disclosure and are not used to interpret the present disclosure in a limited manner. The description herein is merely exemplary and is not intended to limit the claims or applications of the present disclosure in any way.

The present embodiment will be described in detail enough so that those skilled in the art can carry out the present disclosure. It should be understood that other implementations and embodiments may be made, and configurations and structures may be modified and various elements may be replaced without departing from the scope and spirit of the technical idea of the present disclosure. Therefore, the following description should not be interpreted as being limited thereto.

[First Embodiment]

In the first embodiment, a cause of prolongation of a blood clotting time is estimated using features extracted from waveforms (WaveNor0, WaveNor1, and WaveNor2) obtained by normalizing Wave0, Wave1, and Wave2 obtained by waveform fitting based on a normalization based on the reaction constants (NRC) method to be described later.

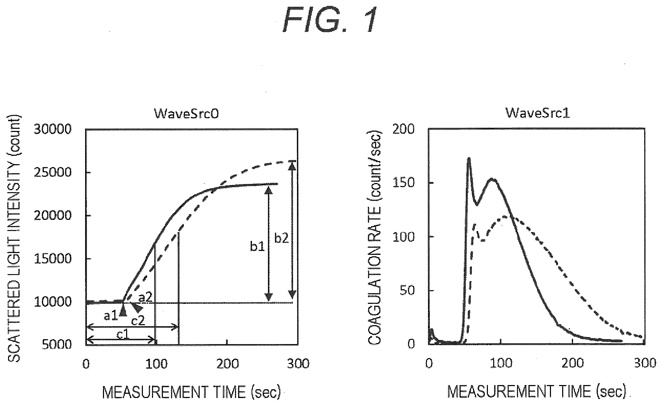

samples used for acquiring clot waveforms contain a plurality of blood coagulation factors. A difference in concentration of each factor results in a difference in coagulation reaction rate, leading to diversity (variation) in blood clotting time. In this field, a first derivative of the clot waveform is treated as a coagulation rate, and a second derivative of the clot waveform is treated as a coagulation acceleration. Even in the same sample group, clot waveforms or coagulation rate-related waveforms do not match, making it difficult to visually recognize the feature of the sample group. As an example, FIG. 1 illustrates clot waveforms of two FVIII-deficient samples (hereinafter, these waveforms will be appropriately referred to as "WaveSrcO"), and first waveforms corresponding to first derivatives of the two clot waveforms (WaveSrc0) (hereinafter, these waveforms corresponding to the first derivatives of the clot waveforms will be appropriately referred to as "WaveSrc1"). In WaveSrc0, the rise time in the waveform (arrowhead positions a1 and a2), the total amount of change in light amount (arrow width b1 and b2), the blood clotting time (e.g., times c1 and c2 at which the changes in light amount (scattered light intensity (count) in FIG. 1) is 50%), and the like are different for each sample. The total amount of change in light amount depends on a fibrinogen concentration, which is one of the blood coagulation factors. The blood clotting time is influenced by the rate of coagulation reaction, and it is obvious in a hemophilia sample that the more severe the symptom, the longer the blood clotting time. In WaveSrc1 as well, there is a difference in height and width of waveform for each sample. It is also an experimental fact that a waveform feature such as a coagulation rate (change in light amount per unit time (scattered light intensity (count)) decreases in inverse proportion to the blood clotting time as the blood clotting time increases, and is different for each sample influenced by the difference in blood clotting time. However, it is necessary to find a common feature between these two samples, which are identical in that both of them are FVIII-deficient samples, and to classify the two samples into the FVIII-deficient sample group.

The inventors have considered that it is effective to suppress the influence of variations in blood clotting time inside each sample group in order to make apparent a feature common to the same sample group. Based on this idea, in the present disclosure, normalization is performed based on the NRC method for simultaneously normalizing not only a light amount axis but also a time axis. Thereby, it has been found that a waveform feature of the same sample group can be made apparent by converging WaveSrc0 of the same sample group, WaveSrc1 obtained by before-after differentiation processing on WaveSrc0, and WaveSrc2 obtained by before-after differentiation processing on WaveSrc1, and a difference in waveform shape between sample groups can be made apparent when waveforms are compared between the sample groups. A specific standardization method and the like will be described in detail later in the section (Sample Identification Program). WaveSrc2 is a waveform corresponding to the first derivative of WaveSrc1 (corresponding to the second derivative of the clot waveform) .

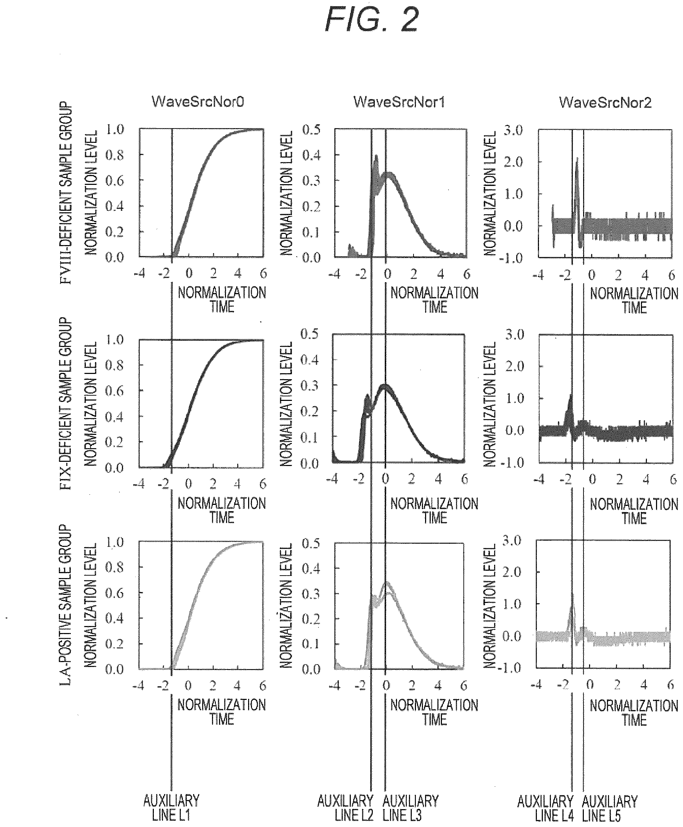

FIG. 2 illustrates WaveSrcNor0, WaveSrcNor1, and WaveSrcNor2, which are waveforms obtained by normalizing WaveSrc0, WaveSrc1, and WaveSrc2 for each of a FVIII-deficient sample group, a FIX-deficient sample group, and a lupus anticoagulant (LA)-positive sample group. It can be seen that the waveforms generally converge in the same sample group in any graph. When the waveforms of the same type (e.g., WaveSrcNor0) were compared between the sample groups, a shape difference was observed. For example, in WaveSrcNor0, there was a difference in rising position in waveform between the sample groups. The auxiliary line L1 was arranged to be located at the center of the rising position in the waveform for the LA-positive group. The rising position in the waveform for the FVIII-deficient sample group was located to the right of the auxiliary line L1, while the rising position in the waveform for the FIX-deficient sample group was located to the left of the auxiliary line L1, and a difference was observed. In WaveSrcNor1 and WaveSrcNor2, there were differences in width between apexes of two ridges and apex height. In both WaveSrcNor1 and WaveSrcNor2 waveforms, auxiliary lines L2 and L4 were arranged to be located at the center of the peak of the left ridge of the LA-positive group, and auxiliary lines L3 and L5 were arranged to be located at the center of the peak of the right ridge of the LA-positive group. Upon checking the peak positions of the right ridges using the auxiliary lines L3 and L5 as guides, no significant difference was observed between the sample groups. On the other hand, upon checking the peak positions of the left ridges were confirmed using the auxiliary lines L2 and L4 as guides, a difference was observed, with the peak of the FVIII-deficient sample group being located to the right of the auxiliary lines L2 and L4, and the peak of the FIX-deficient sample group being located to the left of the auxiliary lines L2 and L4. Since the differences between these sample groups were larger than the variation within the same sample group, the inventors had come up with the idea of quantifying these differences as features for use in identifying samples.

The samples used for acquiring WaveSrc0 illustrated in FIG. 1 and WaveSrc0 that is source data for generating WaveSrcNor0 illustrated in FIG. 2 are all commercially available products. Specifically, the commercially available products are as follows. The FVIII-deficient samples are Factor VIII Deficient Plasma (manufactured by Precision BioLogic, Inc.), FVIII Deficient Plasma (manufactured by George King Bio-Medical, Inc.), and Factor VIII Deficient Plasma (manufactured by Affinity Biologicals, Inc.). The FIX-deficient samples are Factor IX Deficient Plasma (manufactured by Precision BioLogic, Inc.), FIX Deficient Plasma (manufactured by George King Bio-Medical, Inc.), and Factor IX Deficient Plasma (manufactured by Affinity Biologicals, Inc.). LA-positive samples are Lupus Positive Control and Weak Lupus Positive Control (manufactured by Precision BioLogic, Inc.), and Positive LA Plasma (manufactured by George King Bio-Medical, Inc.).

The sample that is a target of the present disclosure is not particularly limited as long as it is a sample in which a coagulation reaction derived from a subject occurs. A test sample composed of plasma separated from blood acquired from the subject is suitable. The test sample composed of plasma may contain a trace amount of impurities as long as there is no problem in analysis by an automatic analyzer of the present disclosure, such as measurement of clot waveforms. The reagent is not particularly limited as long as it is a reagent for measuring a thromboplastin time (PT) item, an activated partial thromboplastin time (APTT) item, and a fibrinogen (Fbg) item. The device only needs to be able to measure a change in light amount (the light amount is an index including turbidity) of a reaction liquid over time resulting from a coagulation reaction. The reagent used for the measurement of clot waveforms described in the present embodiment was Coagpia APTT-N (manufactured by Sekisui Medical Co., Ltd.). The device was Hitachi Automatic Analyzer 3500 (manufactured by Hitachi High-Tech Corporation).

The present disclosure is characterized not only in that normalization based on the NRC method is performed in order to make a difference in waveform shape apparent, but also in that waveform fitting is performed in order to quantify the made-apparent difference in waveform shape while suppressing the influence of noise.

Next, an automatic analyzer for acquiring a clot waveform, a method for acquiring a clot waveform, a sample identification program, and the like will be specifically described.

(Automatic analyzer 100)

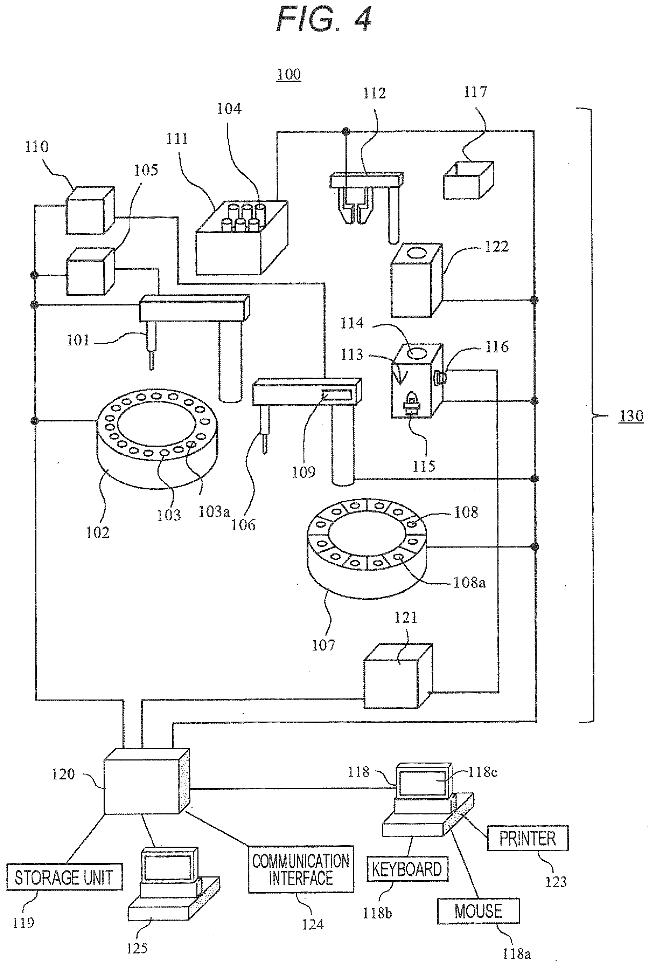

The automatic analyzer 100 used to acquire a clot waveform will be described. FIG. 4 is a diagram illustrating an example of an overall configuration of the automatic analyzer 100 in the first embodiment. Here, basic device operations will be described with reference to FIG. 4, but are not limited to the following example.

As illustrated in FIG. 4, the automatic analyzer 100 mainly includes an analysis unit 130, an operation computer 118, a storage unit 119, and a control computer 120. The analysis unit 130 mainly includes a sample dispensing unit 101, a sample disk 102, a reagent dispensing unit 106, a reagent disk 107, a reaction vessel stock unit 111, a reaction vessel conveyance unit 112, a detection unit 113, and a reaction vessel discard unit 117. The control computer 120 is communicably connected to the analysis unit 130.

The sample dispensing unit 101 aspirates a sample 103a contained in the sample container 103 arranged on the sample disk 102 that rotates clockwise and counterclockwise, and discharges the sample to the reaction vessel 104. The sample dispensing unit 101 executes an operation of aspirating the sample and an operation of discharging the sample by operating a sample syringe pump 105 controlled by the control computer 120.

The reagent dispensing unit 106 aspirates a reagent 108a contained in the reagent container 108 arranged on the reagent disk 107 and discharges the reagent to the reaction vessel 104. The reagent dispensing unit 106 executes an operation of aspirating the reagent and an operation of discharging the reagent by operating a reagent syringe pump 110 controlled by the control computer 120.

A reagent heating unit 109 is built in the reagent dispensing unit 106. The reagent 108a aspirated by the reagent dispensing unit 106 is heated to an appropriate temperature (predetermined temperature) by the reagent heating unit 109 controlled by the control computer 120.

The reaction vessel conveyance unit 112 conveys and installs the reaction vessel 104. The reaction vessel conveyance unit 112 holds and horizontally rotates the reaction vessel 104 to convey the reaction vessel 104 from the reaction vessel stock unit 111 to a reaction vessel installation unit 114 of the detection unit 113 and install the reaction vessel 104.

The detection unit 113 has one or more (one in the present embodiment as an example) reaction vessel installation units 114 for mounting the reaction vessel 104. The detection unit 113 measures a light intensity of a reaction liquid (a mixed liquid of the sample 103a and the reagent 108 a) in the reaction vessel 104 inserted into the reaction vessel installation unit 114. The detection unit 113 is temperature-controlled so that the reaction liquid in the reaction vessel 104 inserted into the reaction vessel installation unit 114 becomes, for example, 37°C. Note that, one detection unit 113 is arranged in the present embodiment. Alternatively, a plurality of detection units 113 may be provided. An example of a detection principle in the detection unit 113 will be described below. Light emitted from a light source 115 is scattered by the reaction liquid in the reaction vessel 104. A detection unit (optical sensor) 116 receives the scattered light scattered by the reaction liquid in the reaction vessel 104. As the light source 115, for example, a halogen lamp or an LED is used. The detection unit (optical sensor) 116 includes a photodiode, etc. The signal received by the detection unit (optical sensor) 116 becomes a light amount value converted into a digital signal by an A/D converter 121, and is input to the control computer 120 as reaction process data (clot waveform data representing a temporal change in detected light amount) and taken into the storage unit 119. An operation of the detection unit 113 is controlled by the control computer 120. Here, the detection unit is a detector using scattering of light. The detection unit may be another detector, e.g., a detector using transmitted light.

The reaction vessel conveyance unit 112 holds the reaction vessel for which the measurement has been completed, and discards the reaction vessel 104 to the reaction vessel discard unit 117.

For the purpose of improving the processing capability, an incubator 122 may be provided without a detector that heats the sample before the measurement start reagent is added.

The control computer 120 is an information processing device having a computer system including a processor, a memory, etc. The control computer 120 not only controls an operation of the automatic analyzer such as dispensing the sample 103a or the reagent 108a, relocating the reaction vessel 104, or discarding the reaction vessel 104, but also calculates a blood clotting time from a light intensity measurement value (clot waveform data) that changes with time according to a coagulation reaction of the reaction liquid, and also estimates an identification of a cause of prolongation of a blood clotting time. The calculated blood clotting time and the identification estimation result of the cause of prolongation of the blood clotting time are output to a display unit 118c and stored in the storage unit 119. These results may be printed out by a printer 123 via the operation computer 118. In addition to programs such as a control program, a measurement program, a calibration curve generation program, a quantification program, and a sample identification program, measured waveform data, a quantification result, a sample identification result, and the like are stored in the storage unit 119 connected to the control computer 120. The various programs are read and executed according to a request input to the operation computer 118 or a request sent from a communication interface 124. An input to the operation computer 118 may be made by touching the display unit 118c, or may be made via a connected keyboard 118b. Alternatively, an input to the operation computer 118 may be made by selecting an item displayed on the display unit 118c with a mouse 118a. The waveform data, the quantification result, the sample identification result, and the like stored in the storage unit 119 are output to the display unit 118c. Alternatively, they are sent to the communication interface 124. Alternatively, they are printed by the printer 123 as necessary. The communication interface 124 is connected to, for example, a network in a hospital, and communicates with a hospital information system (HIS) or a laboratory information system (LIS).

As described above, the estimation of the cause of prolongation of the blood clotting time for the test sample is executed by calling the sample identification program and the waveform data of the test sample stored in the storage unit 119 to the control computer 120. Alternatively, the waveform data of the test sample may be transferred to an analysis computer 125 that executes the sample identification program, and the analysis computer 125 may estimate the cause of prolongation of the blood clotting time for the test sample and display the result on a screen of the analysis computer 125. Alternatively, the waveform data stored in the storage unit 119 may be written out to another external storage medium or the like via the control computer 120 and the operation computer 118, and an independent analysis computer having a sample identification program may read the written-out waveform data and estimate the cause of prolongation of the blood clotting time for the test sample.

(Method for Acquiring Clot Waveform)

Next, an operation of analyzing a blood clotting time item and acquisition of clot waveforms (WaveSrc0) will be described. There are mainly three blood clotting time items: a thromboplastin time (PT) item, an activated partial thromboplastin time (APTT) item, and a fibrinogen (Fbg) item. For any item, first, parameters necessary for analysis are set. In the setting of the parameters, an item to be analyzed, a sample amount, a reagent amount, an output unit, etc. are input. For the PT item or the Fbg item, a calibration method is also input, and calibration is performed as necessary. After the parameters are set, the sample container 103 and the reagent container 108 that contain the sample 103a and the reagent 108a, respectively, are installed on the sample disk 102 and the reagent disk 107, respectively, for analysis. As the reagent 108a, for example, a commercially available reagent for PT measurement, for APTT measurement, or for Fbg measurement can be used.

(Operation of Analyzing PT Item)

An operation of analyzing the PT item will be described. The sample 103a is dispensed into an empty reaction vessel 104 held in the reaction vessel stock unit 111 by the sample dispensing unit 101. The reaction vessel 104 containing the sample 103a is moved to the reaction vessel installation unit 114 of the detection unit 113 by the reaction vessel conveyance unit 112. The reagent 108a is aspirated from the reagent container 108 by the reagent dispensing unit 106, and is heated to an appropriate temperature by the reagent heating unit 109. The temperature at this time is preferably 37°C. The heated reagent 108a is discharged to the reaction vessel 104 already installed in the reaction vessel installation unit 114, with the sample 103a being contained therein. At the same time as the start of the discharge, a change in turbidity over time of the reaction liquid, which is a mixed liquid of the sample 103a and the reagent 108a, is optically measured and WaveSrc0 is obtained.

(Operation of Analyzing APTT Item)

An operation of analyzing the APTT item will be described. The sample 103a is dispensed into an empty reaction vessel 104 held in the reaction vessel stock unit 111 by the sample dispensing unit 101. The reaction vessel 104 containing the sample 103a is moved to the reaction vessel installation unit 114 of the detection unit 113 by the reaction vessel conveyance unit 112. A first reagent contains an activator such as ellagic acid or kaolin, is aspirated from the reagent container 108 by the reagent dispensing unit 106, and is heated to an appropriate temperature by the reagent heating unit 109. The temperature at this time is preferably 37°C. The heated first reagent is discharged to the reaction vessel 104 already installed in the reaction vessel installation unit 114, with the sample 103a being contained therein. The temperature of the mixed liquid of the sample 103a and the first reagent is controlled in the reaction vessel 104. The temperature at this time is preferably 37°C. At this time, the mixed liquid of the sample 103a and the first reagent may be stirred by a stirring unit (not illustrated). A second reagent is a calcium chloride solution, is aspirated from the reagent container 108 by the reagent dispensing unit 106, and is heated to an appropriate temperature by the reagent heating unit 109. The temperature at this time is preferably 37°C. The heated second reagent is discharged to the reaction vessel 104 already installed in the reaction vessel installation unit 114, with the mixed solution of the sample 103a and the first reagent being contained therein. The temperature of the mixed liquid of the sample 103a and the first reagent is controlled for a certain period of time before the discharge of the second reagent, preferably 180 seconds. At the same time as the start of the discharge of the second reagent, a change in turbidity over time of the reaction liquid, which is a mixed liquid of the sample 103a and the first and second reagents, is optically measured and WaveSrc0 is obtained.

(Operation of Analyzing Fbg Item)

An operation of analyzing the Fbg item will be described. The sample 103a is dispensed into an empty reaction vessel 104 held in the reaction vessel stock unit 111 by the sample dispensing unit 101. The reaction vessel 104 containing the sample 103a is moved to the reaction vessel installation unit 114 of the detection unit 113 by the reaction vessel conveyance unit 112. A sample diluted liquid is aspirated from the reagent container 108 by the reagent dispensing unit 106, and is heated to an appropriate temperature by the reagent heating unit 109. The temperature at this time is preferably 37°C. The heated sample diluted liquid is discharged to the reaction vessel 104 already installed in the reaction vessel installation unit 114, with the sample 103a being contained therein. The temperature of the mixed liquid of the sample 103a and the sample diluted liquid is controlled in the reaction vessel 104. The temperature at this time is preferably 37°C. At this time, the mixed liquid of the sample 103a and the sample diluted liquid may be stirred by a stirring unit (not illustrated). The reagent is aspirated from the reagent container 108 by the reagent dispensing unit 106, and is heated to an appropriate temperature by the reagent heating unit 109. The temperature at this time is preferably 37°C. The heated reagent is discharged to the reaction vessel 104 already installed in the reaction vessel installation unit 114, with the mixed liquid of the sample 103a and the sample diluted liquid being contained therein. At the same time as the start of the discharge of the reagent, a change in turbidity over time of the reaction liquid, which is a mixed liquid of the sample 103a, the sample diluted liquid, and the reagent, is optically measured and WaveSrc0 is obtained.

For any item, the measurement is terminated when the coagulation reaction is terminated (when no change in turbidity is observed). Alternatively, after a lapse of a certain time, the measurement is terminated. The certain period of time is, for example, 5 minutes. The optically measured change in turbidity over time is taken into the storage unit 119 via the control computer 120 as a clot waveform.

An example of a clot waveform is as illustrated in FIG. 1, and the horizontal axis represents a measurement time and the vertical axis represents a light amount value. FIG. 1 is an example of a clot waveform of a FVIII-deficient sample (manufactured by Precision BioLogic Inc.) for the APTT item, in which the vertical axis represents a scattered light amount value.

(Sample Identification Program)

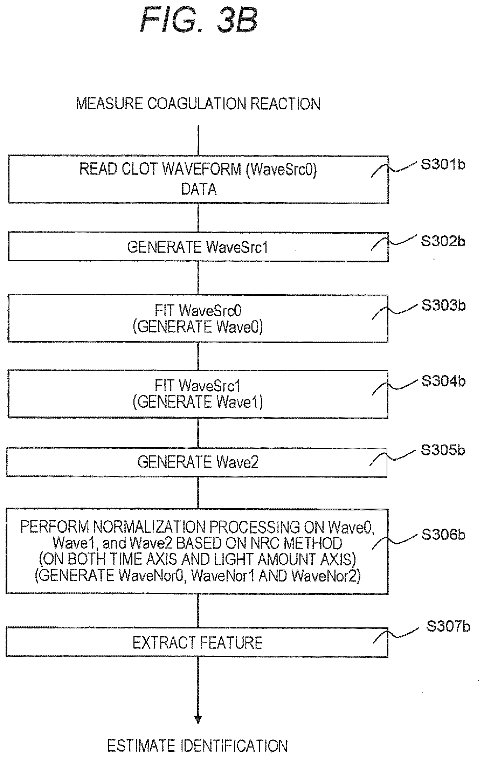

FIG. 3B is a flowchart illustrating a method for estimating a cause of prolongation of a blood clotting time implemented by executing the sample identification program. The computer system of the control computer 120 estimates a cause of prolongation of a blood clotting time in the first embodiment, by executing the sample identification program. A specific description will be given with reference to FIG. 3B. Each step of the flowchart of FIG. 3B may be executed by the control computer 120, the operation computer 118, the analysis computer 125, or a computer on another network. Here, an example in which the computer system of the control computer 120 executes each step will be described.

The control computer 120 reads clot waveform (WaveSrc0) data acquired by measuring a coagulation reaction (S301b in FIG. 3B). The specific analysis operation and the like regarding the acquisition of WaveSrc0 are as described above in the section (Method for Acquiring Clot Waveform).

Next, the control computer 120 generates WaveSrc1 by before-after differentiation processing on WaveSrc0 (S302b in FIG. 3B). WaveSrc1 is obtained by, for example, Formula 1. Here, $\Delta t$ in the following Formula 1 is a coagulation reaction measurement interval, and is, for example, 0.1 seconds.

[Mathematical formula 1]

$$WaveSrc1(n) = (WaveSrc0(n+1) - WaveSrc0(n-1))/2\Delta t$$

Next, the control computer 120 generates a first fitted waveform Wave0 by waveform fitting of WaveSrc0 (hereinafter, a waveform obtained by performing fitting processing on the clot waveform will be referred to as a first fitted waveform, and will be appropriately referred to as "Wave0") (S303b in FIG. 3B), and generates Wave1 by waveform fitting of WaveSrc1 (hereinafter, a waveform obtained by performing fitting processing on the first waveform will be referred to as a second fitted waveform, and will be appropriately referred to as "Wave1") (S304b in FIG. 3B). The formula for fitting WaveSrc0 is preferably a formula based on a sigmoid curve based on the fact that the clot waveform is a sigmoid curve (S-shaped curve). For example, Formula 2 is used. Formula 2 is a formula that follows a clot waveform in which the light amount becomes $Y_{dc}$ after the reagent is discharged.

[Mathematical formula 2]

$$Wave0(t) = \max\left( Y_{dc}, \quad Y0 + \frac{Y1 - Y0}{1 + e^{-\frac{t-T0}{T1}}} \right)$$

In Formula 2 described above, $Y_{dc}$ is a base light amount [count], Y0 is a base scattered light amount [count] of the reaction liquid itself, Y1 is a saturated scattered light amount [count], t is a measurement time [sec], T0 is a time [sec] of (Y0+Y1)÷2, and T1 is a reaction time constant [sec].

Formula 2 assumes fitting of the clot waveform measured by scattered light. In a case where the clot waveform is measured by absorbance, the waveform measured by scattered light is inverted upside down, and thus it is only required to refine the waveform to follow the clot waveform measured by absorbance based on Formula 2.

The formula for fitting WaveSrc1 is preferably a formula that can more reproduce bimodality. For example, the left peak of the bimodality may be represented by a Gaussian distribution, the right peak of the bimodality may be represented by a sigmoid derivative, and the two peaks may be connected to each other by a cubic spline function. In many cases, the shape of WaveSrc1 is bimodal, and this tendency is particularly pronounced in a sample with a prolonged blood clotting time.

Next, the control computer 120 generates Wave2 by before-after differentiation processing on Wave1 (hereinafter, a waveform corresponding to the first derivative of the first fitted waveform will be referred to as a third waveform, and will be appropriately referred to as "Wave2") (S305b in FIG. 3B). Wave2 is obtained by, for example, Formula 3.

[Mathematical formula 3]

$$Wave2(n) = (Wave1(n+1) - Wave1(n-1))/2\Delta t$$

Next, the control computer 120 normalizes both the time axes and the light amount axes of Wave0, Wave1, and Wave2 based on the NRC method (S306b in FIG. 3B). The time axes are normalized by Formula 4. The light amount axes for Wave0, Wave1, and Wave2 are normalized by Formula 5, Formula 6, and Formula 7, respectively. A waveform obtained by normalizing the time axis and the light amount axis of Wave0 is a first normalized waveform, and will hereinafter be appropriately referred to as "WaveNor0". A waveform obtained by normalizing the time axis and the light amount axis of Wave1 is a second normalized waveform, and will hereinafter be appropriately referred to as "WaveNor1". A waveform obtained by normalizing the time axis and the light amount axis of Wave2 is a third normalized waveform, and will hereinafter be appropriately referred to as "WaveNor2". The normalization is normalization based on a reaction constant, and will be referred to as a normalization based on the reaction constants (NRC) method. As the reaction constant used in the NRC method, a numerical value obtained by fitting WaveSrc0 can be used. The waveform fitting is performed in order to quantify the difference while suppressing the influence of noise, and extract the difference as a unique feature.

[Mathematical formula 4]

$$\chi(n) = (t(n) - T0)/T1$$

[Mathematical formula 5]

$$WaveNor0(n) = (Wave0(n) - Y_{dc})/(Y1 - Y_{dc})$$

[Mathematical formula 6]

$$WaveNor1(n) = (Wave1(n)/(Y1 - Y_{dc})) \times T1$$

[Mathematical formula 7]

$$WaveNor2(n) = (Wave2(n)/(Y1 - Y_{dc})) \times T1^2$$

Here, n is the number of data points (n≥1), and the relationship between t(n) and n is t(n)=n/10 [sec]. T0, T1, Wave0(n), $Y_{dc}$, and Y1 are obtained from Formula 2.

Finally, the control computer 120 extracts a feature from the normalized waveforms (S307b in FIG. 3B). The feature should be a numerical value of an observed waveform shape difference for each sample group. Now, FIG. 2 will be referred to back. FIG. 2 illustrates waveforms (WaveSrcNor0, WaveSrcNor1, and WaveSrcNor2) obtained by normalizing the original waveforms (WaveSrc0, WaveSrc1, and WaveSrc2) before waveform fitting is performed. The normalization follows Formulas 4 to 7. In FIG. 2, the original waveforms are normalized, and WaveSrc0, WaveSrc1, and WaveSrc2 are substituted for Wave0(n) of Formula 5, Wave1(n) of Formula 6, and WaveSrc2(n) of Formula 7, respectively.

As described above, for example, in WaveSrcNor0, there was a difference in rising position in waveform. The rises were observed from the sample group for which the normalization time is shortest in the following order of: the FIX-deficient sample group, the LA-positive sample group, and the FVIII-deficient sample group. According to Formula 4, which is a time normalization formula, the distance from the normalization time 0 became longer as the reaction time constant T1 was smaller (that is, the reaction was faster). That is, a difference in reaction rate between the sample groups appeared as a difference in rise timing. FVIII becomes activated FVIII, and its presence enhanced a part of the reaction in the coagulation reaction unit. Thus, it is considered that the reaction time constant increased and the rise timing was delayed in the FVIII-deficient sample group. LA constantly inhibits a coagulation reaction regardless of the activity of the blood coagulation factor because LA binds to phospholipid, which is a scaffold of the coagulation reaction, to inhibit the binding of the coagulation factor. As the degree of inhibition varies depending on the amount and quality (titer) of LA, the rate of coagulation reaction varies, leading to a diversity of reaction time constant T1. Thus, it is considered that the LA-positive sample group was located between the FIX-deficient sample group and the FVIII-deficient sample group. In this way, it is considered that the rise timing reflects the difference derived from the cause of prolongation of the blood clotting time, and it was determined that the feature obtained by quantifying the difference in rise timing was useful. Here, the feature was a vector whose start point was a point where the normalization time of each waveform was 0 and whose end point was a point where the waveform rose.

In WaveSrcNor1, there was a difference between in width between apexes of two ridges and apex height. In the FVIII-deficient sample group, it is considered that the first ridge was larger (higher) because FVIII that was present in a trace amount was used in the initial stage of the reaction and then became insufficient. On the other hand, it is considered that in the FIX-deficient sample group and the LA-positive sample group, since FVIII was sufficiently present, the reaction rate was faster in the later amplification reaction, called positive feedback, than in the initial stage of the reaction, and the second ridge was larger (higher). Focusing on the normalization time, there was a difference in the timing at which the waveform rose for the same reason as WaveSrcNor0, leading to a difference in the normalization time at which the first ridge occurs. It is considered that the difference in the generation time of the first ridge and the difference in heights between the two ridges reflect the difference derived from the cause of prolongation of the blood clotting time, and it was determined that the feature obtained by quantifying these differences was useful. Here, the feature was a vector whose start point was a point where the normalization time was 0 and whose end point was a peak point of the first ridge.

In WaveSrcNor2, differences were observed in height and generation time of first peak, influenced by the waveform difference corresponding to the first derivative. Here, by combining information on the peak height of the first ridge and the normalization time, vector information in which the start point was a point at which the normalization time was 0 and the end point was a peak point of the first ridge was set as the feature.

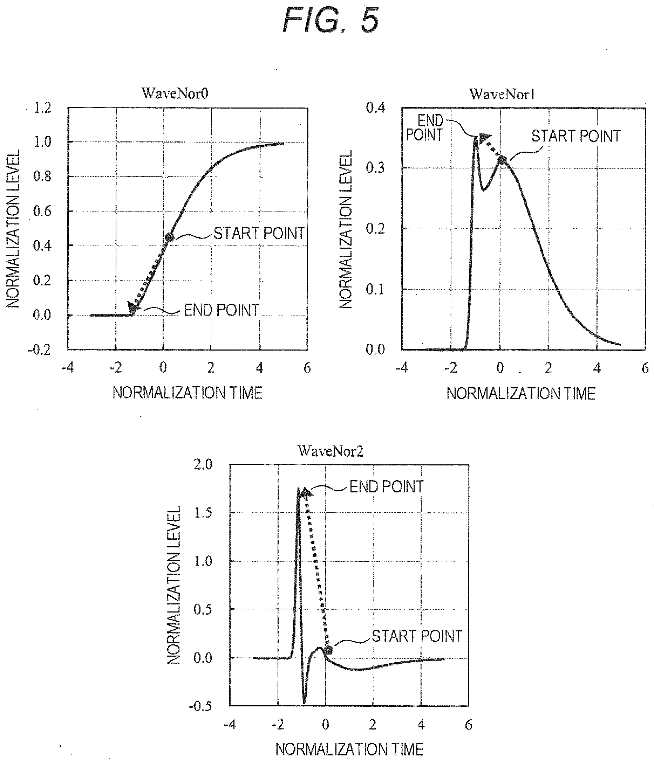

The vector feature is extracted from a waveform (WaveNor0, WaveNor1, WaveNor2) obtained by normalizing a fitted waveform (Wave0, Wave1, Wave2) in order to suppress the influence of noise and for quantification. FIG. 5 illustrates WaveNor0, WaveNor1, and WaveNor2, which are waveforms obtained by normalizing Wave0, Wave1, and Wave2, and feature vectors. The horizontal axis represents a normalization time, and the vertical axis represents a normalization level. The feature vectors of WaveNor0, WaveNor1, and WaveNor2 are vectors 0, 1, and 2, respectively. The x component and the y component of each vector were set as features (X, Y) for each waveform. Here, the x component of WaveNor0 was devised so that the difference in waveform shape of the sample group was emphasized by subtracting TO/T1. The features (X, Y) of WaveNor0, WaveNor1, and WaveNor2 are expressed as (vec0_x, vec0_y), (vec1_x, vec1_y), and (vec2_x, vec2_y), respectively.

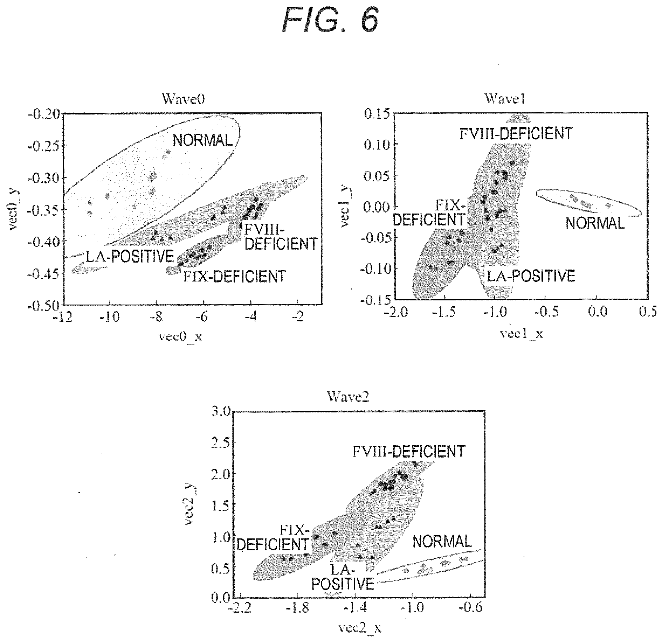

Finally, an extracted feature of a test sample is compared with the features of the samples whose background (the cause of prolongation of the blood clotting time) has been known, and an identification of a cause of prolongation of a blood clotting time for the test sample is estimated. The features (X, Y) extracted from the samples for which the cause of prolongation of the blood clotting time has been known are independently used, and a probability density distribution is given to a feature population (cluster) for each sample group in a two-dimensional map on which these features are plotted. For example, the probability density distribution is based on a mixed Gaussian distribution model. FIG. 6 illustrates examples of two-dimensional maps including features extracted from WaveNor0, WaveNor1, and WaveNor2, which are normalized waveforms. The maps are expressed as Wave0, Wave1, and Wave2. The features are extracted by analyzing waveforms obtained from samples for which the cause of prolongation of the blood clotting time has been known. Each ellipse illustrated for a population (cluster) of features of each of the sample groups (FVIII-deficient, FIX-deficient, LA-positive, normal) represent a spread of feature data. The size of each ellipse was set to $4\sigma$ ($\sigma$: standard deviation in each direction, called Mahalanobis distance in two or more dimensions) from the center, so that the ellipses for normal samples and abnormal samples do not overlap. These ellipses are represented by probability densities based on the Gaussian distribution. In FIG. 6, a region that does not belong to any ellipse indicates that it does not belong to any sample group in the range of given data, and indicates that it is a novel clot waveform that occurs when two or more types of test samples are artificially mixed, depending on the medication status of a patient under treatment, or the like. This region is defined as an "unknown" group. The ellipses for the abnormal samples overlap each other in any classifier, and a boundary therebetween can be set to a certain value of a belonging probability calculated according to the probability density distribution.

In FIG. 6, a case where the boundary is a belonging probability of 50% is shown. The abnormal samples used here are the commercially available products described above. The normal samples were also substituted with commercially available products. Specifically, the normal samples were Normal Reference Plasma and Pooled Normal Plasma (manufactured by Precision BioLogic, Inc.), and Factor Assay Control Plasma and Borderline Factor Assay Control Plasma (manufactured by George King Bio-Medical, Inc.).

The features extracted from WaveNor0, WaveNor1, and WaveNor2 of the test sample are applied to the three two-dimensional maps, and belonging probabilities for the test sample in each map (probabilities for determining which sample group the test sample belong to) are calculated. Based on the calculated belonging probabilities, an identification of the test sample is estimated. For example, the identification of the test sample may be estimated as the sample group having the highest belonging probability. Alternatively, the identification of the test sample may be estimated as the sample group having the highest average belonging probability between the maps. Alternatively, the identification of the test sample may be estimated as the sample group having the highest weighted-average belonging probability between the maps.

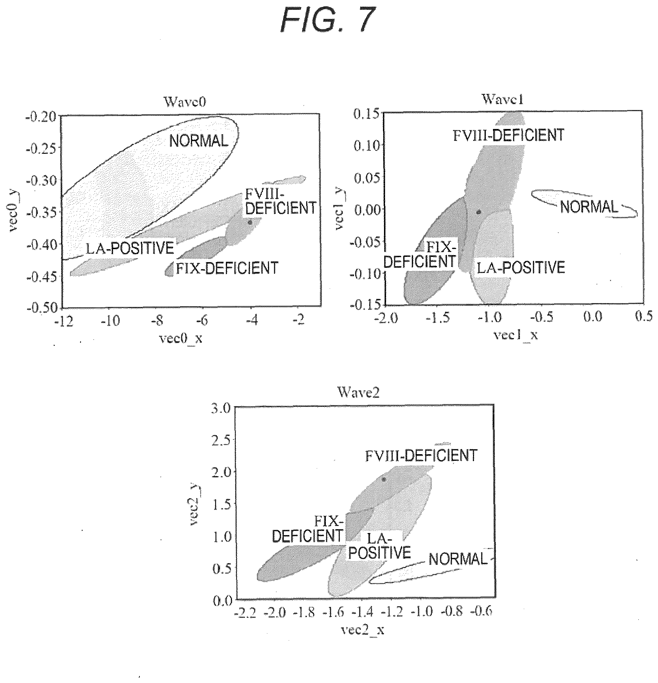

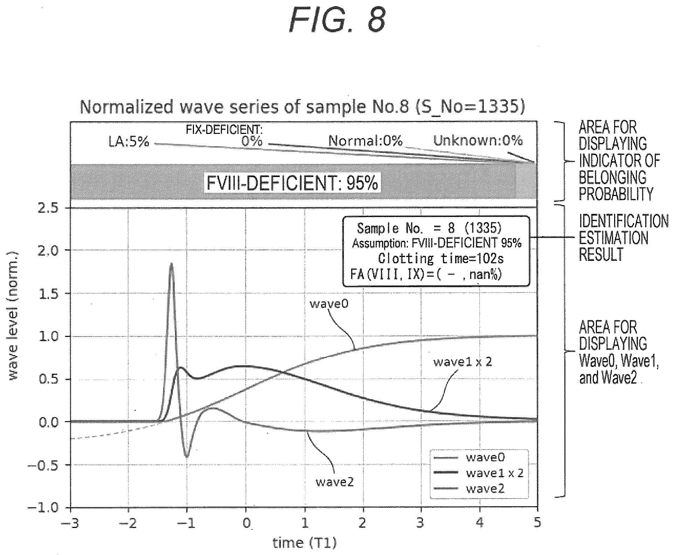

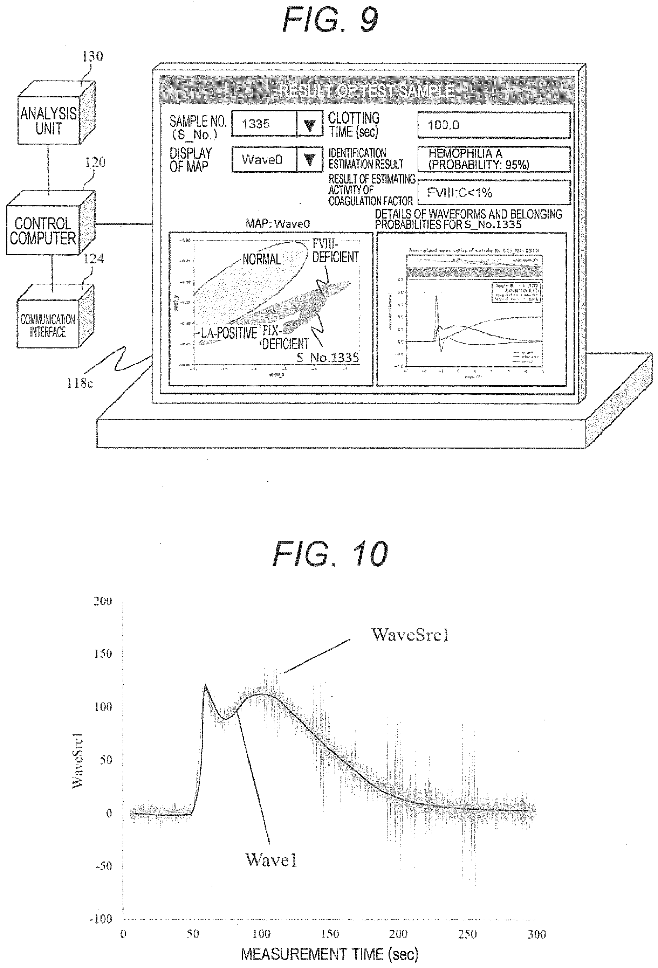

An example of display of the identification estimation result of the test sample is illustrated in FIGS. 7 and 8. FIG. 7 is a diagram illustrating an example of where the features of the test sample are plotted in the two-dimensional maps created from the known features of samples. FIG. 8 is a diagram illustrating WaveNor0, WaveNor1, and WaveNor2 (indicated as Wave0, Wave1×2, and Wave2 in the diagram) of the test sample, an indicator showing probabilities that the test sample belongs to the FVIII-deficient sample group, the FIX-deficient sample group, the LA-positive sample group, the normal sample group, and the unknown sample group, and an identification estimation result. FIG. 9 illustrates a relationship between the analysis unit 130, the control computer 120, and the communication interface 124, and an example in which the result of the test sample is displayed on the display unit 118c. On the display unit 118c, a field for selecting the sample number (S_No.) of the test sample of which the result is to be displayed and the two-dimensional map is displayed. It is preferable that the blood clotting time of the selected sample number, the identification estimation result, and the estimation result of the activity of the blood coagulation factor may be displayed on the display unit 118c. It is not always necessary to display the estimation result of the activity of the blood coagulation factor, but it is better to do so. For example, blood coagulation factor VIII deficiency is a state in which the activity of the blood coagulation factor VIII has a value of less than 1%. Blood coagulation factor IX deficiency is a state in which the activity of the blood coagulation factor IX has a value of less than 1%. In FIG. 9, the selected map and the feature of the test sample plotted in the map are shown on the lower left side of the screen. By performing such display, it is possible to intuitively express the cause of prolongation of the blood clotting time for the test sample with high visibility. Meanwhile, it is also important to quantitatively indicate the cause of prolongation. In FIG. 9, "hemophilia A (probability: 95%)" is displayed as the "identification estimation result". In addition, as shown on the lower right side of the screen of FIG. 9, details (e.g., the contents of FIG. 8) of waveforms and belonging probabilities for the sample of the selected sample number may be displayed, and the waveforms may be visualized and an indicator displaying the details of the belonging probabilities may be shown.

Note that, in the first embodiment, preprocessing such as smoothing processing may be appropriately performed, for example, after reading of WaveSrc0 (S301b in FIG. 3B) and/or after generation of WaveSrc1 (S302b in FIG. 3B). Preprocessing (first filter processing for removing noise of WaveSrc0 and second filter processing for removing noise of WaveSrc1) means filter processing for noise reduction for each waveform. This processing step is not necessarily required. However, in order to more accurately fit a waveform, it is preferable to perform the preprocessing before the fitting of the waveform. Specific examples of the filter processing include N-point moving average processing (N is a positive integer and an odd number, and $3 \leq N \leq$ the number of measurement points) and smoothing processing performed by selecting a minimum value in a certain section. With respect to a variation in blood clotting time value, in order to avoid loss of original waveform shape, a T1 point moving average using a reaction time constant as a fitting parameter according to Formula 2 may be obtained. At this time, for example, one decimal place of the value of T1 may be rounded off to an integer, and in a case where the integer is an even number, 1 may be added or 1 may be subtracted to be used as an odd number.

(Example of Result of Executing Sample Identification Program)

As a result of estimating the identification of the test sample in the sample identification program, the test sample was classified as the FVIII-deficient sample group, the FIX-deficient sample group, and the LA-positive sample group in 85.7% (=12/14*100%), 100% (=9/9*100%), and 66.7% (=4/6*100%), respectively. The probability is 86% (=25/29*100%) as a whole. It was confirmed that the normal samples could be classified by 100% (=10/10*100%), and the normal samples were not erroneously determined as the FVIII-deficient sample group, the FIX-deficient sample group, or the LA-positive sample group. The test sample used is a product different in lot from the commercially available products described above.

(Effects of First Embodiment)

FIG. 10 illustrates examples of WaveSrc1 and Wave1. It can be seen that the waveform of WaveSrc1 includes noise. As a method for removing noise, preprocessing such as smoothing processing has been disclosed. However, excessive processing has a problem that it leads to loss of waveform shape. Insufficient processing has a problem that when a certain feature is extracted as a numerical value, a plurality of values exist and the feature is not uniquely determined. The fact that, when a feature inherent in a clot waveform is estimated as a numerical value, there are a plurality of values and the feature is not uniquely determined means that, for example, in a case where it is desired to extract the maximum value of the left one of the two ridges in FIG. 10, the raw data contains noise and has a plurality of maximum values at different times, and the maximum value is not determined by one point. The method of fitting a waveform to extract a feature solves such a problem by determining a waveform model parameter from an observed waveform including noise, for example, such that an RMS error is minimized, and then quantifying a feature inherent in the clot waveform from the fitted waveform including no noise.

The cause of prolongation of the blood clotting time is estimated using the extracted feature. Specifically, features are extracted using samples for which the causes of prolongation of the blood clotting time has been known, and a probability density distribution is given to a population (cluster) of features for each sample group in a two-dimensional map on which these features are plotted. The feature of the test sample is applied to the two-dimensional map to calculate a belonging probability for the test sample (a probability for determining which sample group the test sample belongs to). The cause of prolongation of the blood clotting time for the test sample can be estimated intuitively with high visibility according to the belonging probability.

The present disclosure presents a novel method for solving the above-described problems of the conventional art in that a feature inherent in a clot waveform is extracted as a numerical value via a fitting model, and a feature for each cause of prolongation of the blood clotting time is made apparent by performing normalization processing by an NRC method based on a reaction constant.

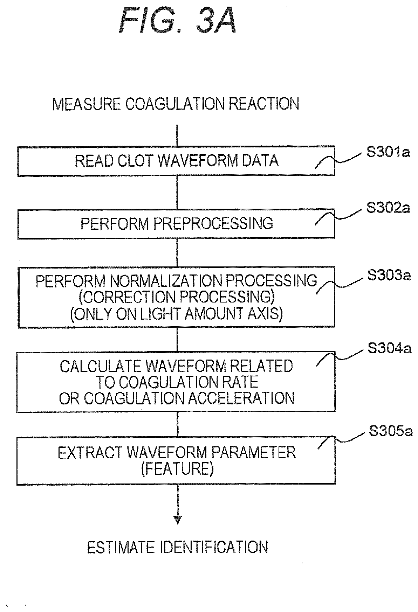

Here, FIG. 3A illustrates an example of a sample identification flow according to a comparative example. In the example of the sample identification flow according to the comparative example (FIG. 3A), first, clot waveform data acquired by measuring a coagulation reaction is read (S301a in FIG. 3A). Next, preprocessing such as smoothing processing is performed (S302a in FIG. 3A). Next, normalization processing (which may also be expressed as correction processing) is performed (S303a in FIG. 3A). The normalization processing is performed only on the light amount axis. Next, a time derivative (which may also be simply expressed as a derivative) or a further time derivative of the waveform data is calculated to calculate a waveform related to a coagulation rate or a coagulation acceleration (S304a in FIG. 3A). Finally, a waveform parameter is extracted as a feature (S305a in FIG. 3A), and for example, the identification of the test sample is estimated from the correlation between the feature of the test sample and the features of the samples for which the cause of prolongation of the blood clotting time has been known. At this time, the preprocessing and the normalization processing on the light amount axis are not necessarily required, and these steps may be skipped. Although not illustrated in the flow, there is also a case where an axis representing a light amount change rate of a waveform related to a coagulation rate or a coagulation acceleration is normalized, and a case where normalization with a maximum value of each waveform as 100% is performed is disclosed.

In contrast, the present disclosure is greatly different from the comparative example in that waveform fitting is performed, both the time axis and the light amount axis are normalized, and identification can be intuitively estimated with high visibility from highly visible and intuitive estimation from the extracted feature. In the example of the sample identification flow according to the first embodiment (FIG. 3B), first, clot waveform (WaveSrcO) data acquired by measuring a coagulation reaction is read (S301b in FIG. 3B). Next, WaveSrc1 is generated by before-after differentiation processing of WaveSrc0 (S302b in FIG. 3B). Next, Wave0 is generated by waveform fitting of WaveSrc0 (S303b in FIG. 3B), and Wave1 is generated by waveform fitting of WaveSrc1 (S304b in FIG. 3B). Next, Wave2 is generated by before-after differentiation processing of Wave1 (S305b in FIG. 3B). WaveSrc1 or Wave1 in the present disclosure corresponds to a waveform related to a coagulation rate in the comparative example. Wave2 in the present disclosure corresponds to a waveform related to a coagulation acceleration in the comparative example. Next, Wave0, Wave1, and Wave2 are normalized based on the NRC method (S306b in FIG. 3B). The normalization based on the NRC method in the present disclosure is normalization based on a reaction constant, and means normalization on both a time axis and a light amount axis. The normalization is performed in relation to Wave0, Wave1, and Wave2 in the present disclosure, rather than normalizing a clot waveform or a waveform related to a coagulation rate or a coagulation acceleration by the maximum value of each waveform when the waveform is normalized on the light amount axis as disclosed in the comparative example. Finally, a feature is extracted from the normalized waveform (S307b in FIG. 3B), and the feature of the test sample is applied to the two-dimensional map created with a probability density distribution using the features extracted from the samples for which the cause of prolongation of the blood clotting time has been known to estimate the identification of the test sample. In the present disclosure as well, preprocessing such as smoothing processing may be appropriately performed, for example, after reading of WaveSrc0 (S301b in FIG. 3B) and/or after generation of WaveSrc1 (S302b in FIG. 3B).

As a main inventive step of the present disclosure, normalization is performed in the time axis direction according to a reaction constant measured using a fitting model of WaveSrc0, and instead of a time derivative of a clot waveform (simply described as a derivative) disclosed in PTL 1 to PTL 4 and NPL 1, a difference using a time constant included in the reaction constant (hereinafter referred to as a reaction time constant difference) is introduced, making it possible to estimate the cause of prolongation of the blood clotting time while reducing the influence of the severity of the symptom expressed by the length of the blood clotting time.

[Second Embodiment]

In the second embodiment, an identification of a clot waveform (limited only to an example using WaveSrc0 from the viewpoint of unity of invention) using a neural network will be described.

Here, an embodiment related to a method for estimating a cause of prolongation of a blood clotting time by a neural network using clot waveform data as an input based on the time axis normalization method according to the present disclosure will be described. As described above, the estimation of the cause of prolongation of the blood clotting time by the neural network directly using the clot waveform data is not disclosed as a set of techniques that can be easily reproduced by a person skilled in the art. Thus, the following three disclosure points form the essence of the present embodiment.

(1) A preprocessing technique suitable for a neural network is disclosed because a measured clot waveform is variable-length data.

(2) To prove that clustering of clot waveforms by the neural network is practical, an identification accuracy of >90% is demonstrated.

(3) In order to easily reproduce the above-described neural network and to facilitate expansion such as application to measurement data of various devices and application to samples of patients under treatment, configuration parameters such as the number of nodes and the number of hidden layers, and hyper parameters relating to learning conditions such as the number of epochs and the number of batches are presented.

(1) Disclosure of Preprocessing

First, a preprocessing technique suitable for a neural network will be described.

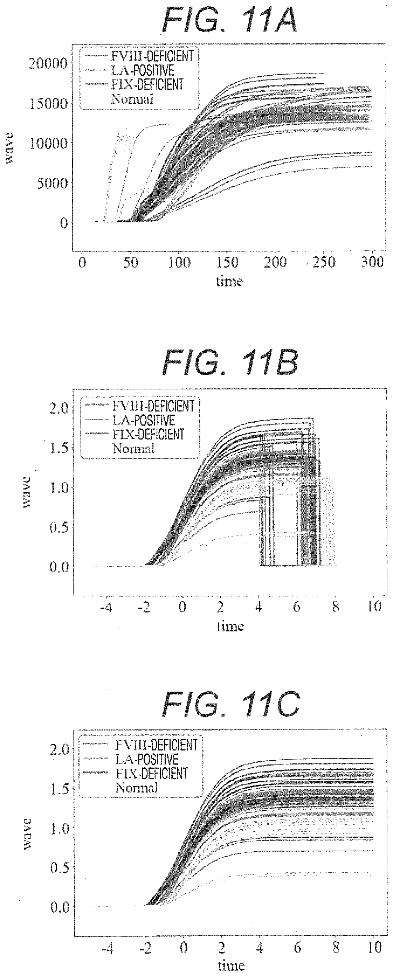

FIGS. 11A to 11C are experimental results showing clot waveforms (WaveSrc0) of commercially available samples and clot waveforms subjected to preprocessing to be suitable for the neural network. FIG. 11A is WaveSrc0 of commercially available samples. Here, 103 waveforms including the FVIII-deficient sample group (46 waveforms), the FIX-deficient sample group (22 waveforms), the LA-positive sample group (6 waveforms), and the normal sample group (29 waveforms) were used. The measurement device used for measurement was Hitachi Automatic Analyzer 3500 (manufactured by Hitachi High-Tech Corporation) . The vertical axis represents a count value of quantized data by the A/D converter corresponding to a scattered light intensity.

FIG. 11B illustrates waveforms obtained by processing WaveSrc0 using a zero padding method, which is known in the field of image and voice recognition, as preprocessing for the neural network. Here, the time axis was normalized according to Formula 4 for normalizing the time axis using a reaction time constant and a response time, which is a main inventive step of the present disclosure, and the clot waveform was resampled by linear interpolation to 501 equally spaced data points in the range of $-5\tau$ to $+10\tau$. The vertical axis is normalized such that the value at the start of measurement is 0 and the change in scattered light intensity of 10,000 counts is 1. This normalization is for adaptation to a general sigmoid or a hyperbolic tangent as an activation function of a neural network. The normalization used here is for using an amount of change in scattered light intensity as a feature, and the normalization is performed according to Formula 8, which is different from Formula 5 described above.

[Mathematical formula 8]

$$WaveO_{Norm.}(n) = (WaveO(t(n)) - Ydc)/10000$$

For the time range of $-5\tau$ to $+10\tau$, no data present due to the termination of the measurement is complemented with data "0" to align the data lengths.

FIG. 11C illustrates waveform data in a case where the fitting model is used to complement insufficient data. By using parameters $\tau$, T0, Y0, and Y1 obtained by a fitting model of Formula 2, it is easy to obtain continuous numerical data even at the time after the measurement is completed. In this sense, it can be said that the introduction of the fitting model has high affinity for identifying a clot waveform using the neural network. As a simple method equivalent thereto, insufficient data can be complemented by copying the value of the last measured data point. FIG. 11C is different from FIG. 11B in that the data indicating the scattered light intensity after the measurement end point is smooth. As the preprocessing used for the neural network, the preprocessing illustrated in FIG. 11B or 11C, in which the data length is fixed, is suitable.

(2) Configuration of Neural Network and Proof of Practical Performance

Next, a configuration of the neural network will be described.

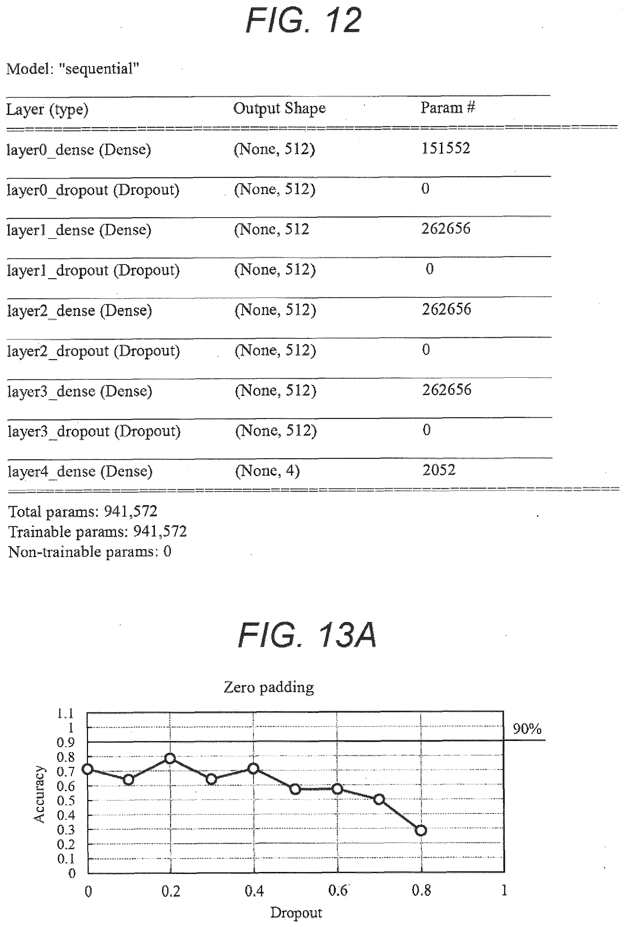

FIG. 12 illustrates a structure of a neural network according to the present disclosure. In FIG. 12, a summary using Keras included in TensorFlow, which is generally used to study a neural network, is illustrated. As can be seen from FIG. 12, the neural network according to the present disclosure can provide the practical performance described above with the configuration in which all layers are densely coupled with 4 hidden layers and 512 nodes. The number of epochs of learning is 10,000, the batch size is 32, and a hyperbolic tangent is used as an activation function. With respect to the activation function, it should be easily understood by a person skilled in the art that equivalent identification performance can be obtained even if the sigmoid is used instead of the hyperbolic tangent.

Hereinafter, description will be made, assuming that hyper parameters are constant: the number of epochs is 10,000 and the batch size is 32. The clot waveform data of commercially available samples is divided into 60% for use in learning, 20% for use in validation, and 20% for use in test, and cross entropy is used as a loss function. The reason for dividing the data into three parts will be described. Since parameters such as weights constituting the neural network are automatically updated with respect to the learning data, the hyper parameters such as the number of hidden layers, the number of nodes, the dropout, the number of epochs, and the batch size are appropriately selected so as to minimize the cross entropy of the validation data. In order to objectively evaluate the performance of the neural network defined by the parameters, it is necessary to evaluate the cross entropy and the identification accuracy using test data that is not used in the above-described procedure.

FIGS. 13A to 13C are experimental results each showing a relationship between a learning result and a dropout. Here, the results are shown for the number of nodes = 256 and the number of hidden layers = 3. FIG. 13A illustrates a result in a case where clot waveforms of which the data lengths are aligned by the zero padding method illustrated in FIG. 11B. It can be seen that even if the dropout amount, which is effective in suppressing over-learning, is changed, the accuracy in identifying test data does not exceed 90%.

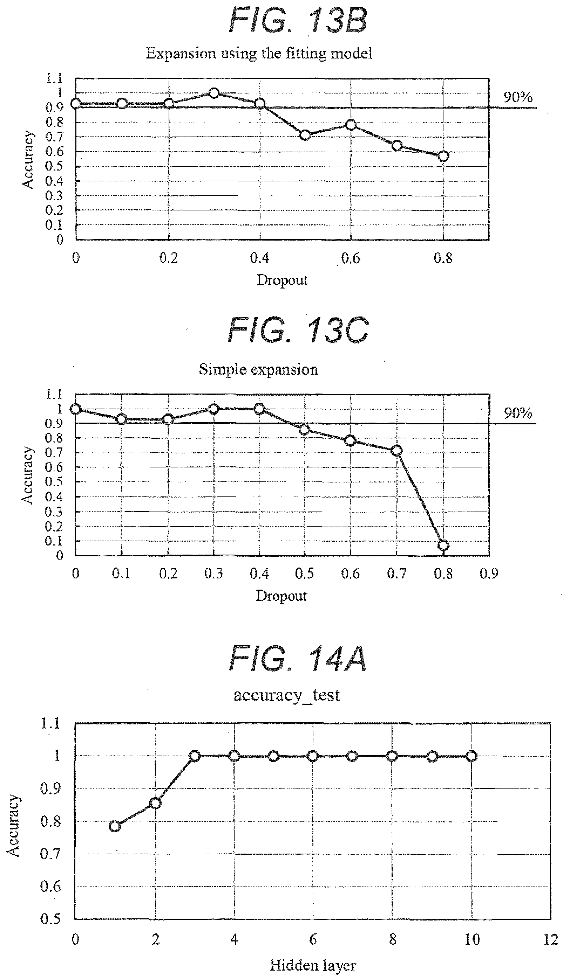

FIG. 13B illustrates a result in a case where clot waveforms preprocessed by data extrapolation complementation using the fitting model illustrated in FIG. 11C are used. As can be seen from FIG. 13B, it can be seen that the accuracy of 90% or more, which is a standard of practical identification accuracy, can be obtained under the condition that the dropout amount is 0.4 or less. This proves that the sample identification method based on the analysis of the clot waveform using the preprocessing method according to the present disclosure and the configuration of the neural network is an identification method that can be easily reproduced and expanded by a person skilled in the art.

FIG. 13C illustrates a result in a case where clot waveforms complemented by copying the last measured data point to align their data lengths are used. Similarly to what is described above, it can be seen that the identification accuracy is 90% or more under the condition that the dropout amount is 0.4 or less. This method has an advantage that less computational effort is required as compared with that of the complementation method illustrated in FIG. 13B. When the amount of sample data is further increased or when noise is mixed during measurement for a certain reason, it can be said that the data extrapolation complementation using the fitting model is superior in terms of stability of identification performance. Hereinafter, unless otherwise specified, the preprocessing is performed by the data extrapolation complementation using the fitting model.

(3) Presentation of Configuration Parameters Such as Number of Nodes and Number of Hidden Layers, and Hyper Parameters Related to Learning Conditions Such as Number of Epochs and Number of Batches

Next, ranges of parameters suitable for implementing the present disclosure will be described.

The number of epochs, the dropout, and the like suitable for training the neural network are as described above. Here, the number of nodes and an example will be described as numerical values indicating the configuration of the neural network.

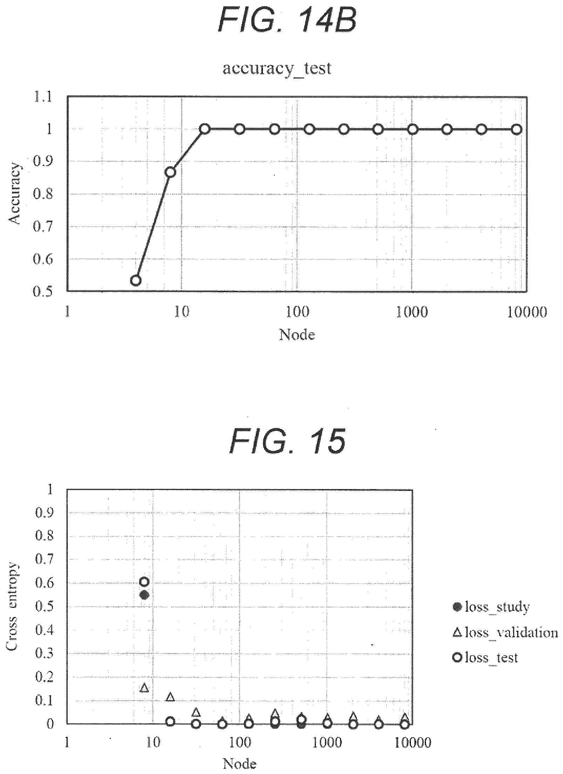

FIGS. 14A and 14B illustrate experimental results showing ranges of hyper parameters in which a classifier that combines the preprocessing method according to the present disclosure and a neural network exhibits practical performance. FIG. 14A illustrates a relationship between the number of hidden layers and an identification accuracy. Here, the number of nodes is 1024, and the dropout is 0.25. As can be seen from FIG. 14A, it can be seen that the identification method according to the present disclosure can obtain a practical identification accuracy of 90% or more when the number of hidden layers is 3 or more.

FIG. 14B illustrates an experimental result showing a relationship between the number of nodes and an identification accuracy. Here, the number of hidden layers is three. As can be seen from FIG. 14B, it can be seen that the identification method according to the present disclosure can obtain a practical identification accuracy of 90% or more when the number of nodes is 16 or more. These results show that the identification method according to the present disclosure can obtain a practical identification accuracy by configuring a neural network in which the number of hidden layers is 3 or more and the number of nodes is 16 or more.

FIG. 15 illustrates an experimental result showing that the identification method using the preprocessing method according to the present disclosure and the neural network realize sufficient suppression of over-learning. Here, a relationship between the number of nodes and a cross entropy for learning, validation, and test samples is shown. In the field of voice and face recognition, where neural networks are widely used, the cross entropy is about 0.1 to 0.3. In contrast, in the identification method according to the present disclosure, in a case where the number of nodes is 16 or more, the cross entropy is about 0.1 or less, and it can be seen that the cross entropy values are substantially equal between the learning sample group, the validation sample group, and the test sample group. This result shows that over-learning can be sufficiently suppressed by the configuration of the neural network presented in the present disclosure.

As described above, according to the present disclosure, a cause of prolongation of a blood clotting time can be estimated with a practical identification accuracy by a neural network having 3 or more hidden layers and 16 or more nodes after preprocessing a clot waveform obtained from a sample to have a predetermined data length using a reaction time constant and a response time quantified by a fitting model. Needless to say, since the classifier based on the neural network outputs a probability of belonging to each cluster as a cause of prolongation of a blood clotting time, it is obvious that the classifier based on the neural network presented here can also display the belonging probabilities exemplified in FIG. 8.

By applying the NRC method to the waveform data used for learning, it is possible to further improve the identification accuracy.

(Effect of Second Embodiment)

By performing suitable preprocessing on a clot waveform having a variable data length, it is possible to estimate a cause of prolongation of a blood clotting time with practical accuracy using the neural network.

[Third Embodiment]

In the third embodiment, application to a cross mixing test will be described.

An identification of a cause of prolongation of a blood clotting time using a clot waveform obtained by measuring a sample collected from a subject has been described above. Meanwhile, in a case where the blood clotting time of the sample collected from the subject is prolonged, a cross mixing test is recognized as a typical method for identifying the cause of the prolongation of the blood clotting time. In the cross mixing test, blood clotting times of a plurality of mixed plasma samples obtained by mixing the sample and a normal sample at predetermined ratios are measured, and the cause of the prolongation of the blood clotting time is screened from a shape of a graph in which the blood clotting times are plotted with respect to the ratios in which the sample is mixed. According to the present disclosure, from a series of clot waveforms acquired from the plurality of mixed plasma samples, not only dependence of the blood clotting time on the mixing ratio but also a plurality of features according to the causes of prolongation of the blood clotting times included in the series of clot waveforms and probabilities of belonging to the respective causes of prolongation can be obtained. Therefore, the sample identification method according to the present disclosure can be easily applied to clot waveform data acquired by a cross mixing test, and it is expected to improve the identification accuracy as compared with that in the related art. When focusing on the affinity with the conventional method, for example, it can be said that a method is preferable in which the dependence of the blood clotting time on the mixing ratio is quantified, and simultaneously, blood coagulation factor VIII deficiency and blood coagulation factor IX deficiency are quantified from the clot waveforms of samples collected from the subject, and they are mapped in a two-dimensional space.

[Fourth Embodiment]

In the fourth embodiment, the feature of the clot waveform obtained by the normalization processing using the NRC method based on the reaction constant is expanded.

That is, in the fourth embodiment, features are extracted from not only a first normalized waveform (WaveNor0), a second normalized waveform (WaveNor1), and a third normalized waveform (WaveNor2), but also waveform data obtained by performing a nonlinear operation thereon. A cause of prolongation of a blood clotting time for a test sample is estimated based on the information on a distribution of features for each cause of the prolongation of blood clotting time prepared in advance and the extracted features.

The method for estimating a cause of prolongation of a blood clotting time for a test sample in the fourth embodiment includes:

(A) acquiring a clot waveform showing a change in light amount over time according to a coagulation reaction of a reaction liquid generated by mixing a test sample composed of plasma separated from blood acquired from a subject and a reagent;

(B) acquiring a first waveform by performing differentiation processing on the clot waveform;

(C) acquiring a first fitted waveform by performing fitting processing on the clot waveform;

(D) acquiring a second fitted waveform by performing fitting processing on the first waveform;

(E) acquiring a third waveform by performing before-after differentiation processing on the second fitted waveform;

(F) acquiring a first normalized waveform, a second normalized waveform, and a third normalized waveform by normalizing the first fitted waveform, the second fitted waveform, and the third waveform, respectively, on a light amount axis and a time axis;

(G) extracting a feature from at least one of the first normalized waveform, the second normalized waveform, the third normalized waveform, or waveform data obtained by performing a nonlinear operation thereon; and

(H) estimating a cause of the prolongation of blood clotting time for the test sample based on information on a distribution of features for each cause of prolongation of the blood clotting time prepared in advance and the feature extracted from the clot waveform of the test sample.

Hereinafter, in the present invention, the FVIII-deficient sample group will be referred to as "FVIII-deficient", the FIX-deficient sample group will be referred to as "FIX-deficient", the LA-positive sample group will be referred to as "LA", and the normal sample group will be referred to as "Normal" in order to simplify the drawings and help understanding of the technology. Here, a clot waveform measured by Hitachi Automatic Analyzer 3500 (manufactured by Hitachi High-Tech Corporation) using a commercially available sample is used. The measurement reagent is Coagpia APTT-N (manufactured by Sekisui Medical Co., Ltd.). As commercially available samples, in addition to the commercially available products described in the first embodiment, Control P-N I for Coagpia (manufactured by Sekisui Medical Co., Ltd.) and Coagtrol N (manufactured by Sysmex Corporation) were used as normal samples.

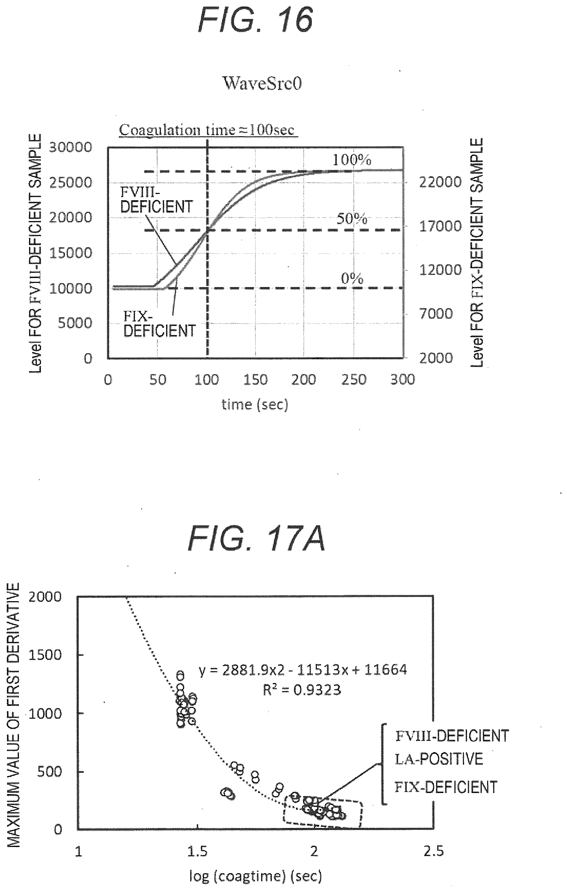

FIG. 16 illustrates a comparison of WaveSrc0 of one representative FVIII-deficient sample and WaveSrc0 of one representative FIX-deficient sample. As can be seen from FIG. 16, the blood clotting times of both samples are almost the same, about 100 seconds. On the other hand, it can be seen that the FVIII-deficient sample has a slower convergence of reaction (takes a longer time until the scattered light intensity reaches the saturation value from the initial value) as compared with the FIX-deficient sample. This shows a difference in influence on the reaction process between both deficiency factors. This feature is that, between the two sample, for example, in Formula 2, TO is approximately equal, and T1 is FIX-deficient < FVIII-deficient. In the present disclosure, (T0/T1) was subtracted from vec0_x.

A time until the level reaches 50% when the initial value of the scattered light intensity is 0% and the saturation value of the scattered light intensity is 100% is calculated as a clotting time. The method of calculating the blood clotting time is not limited to this method. For example, a time until a certain level other than 50% is reached may be calculated as a blood clotting time.

FIG. 17A illustrates an experimental result showing a relationship between a maximum value of a first derivative of a clot waveform and a log clotting time. The maximum value of the first derivative corresponds to an index |min1| described in PTL 1. As illustrated in FIG. 17A, the R2 value obtained by performing least square fitting with a quadratic function was 0.9323. That is, it can be seen that the maximum value of the first derivative has an extremely high correlation with the blood clotting time. It can be seen that maximum values of first derivatives of the FVIII-deficient sample group, the FIX-deficient sample group, and the LA-positive sample group (LA) is distributed in a very small region of FIG. 17A as compared with the total change amount of the index including the normal sample group.

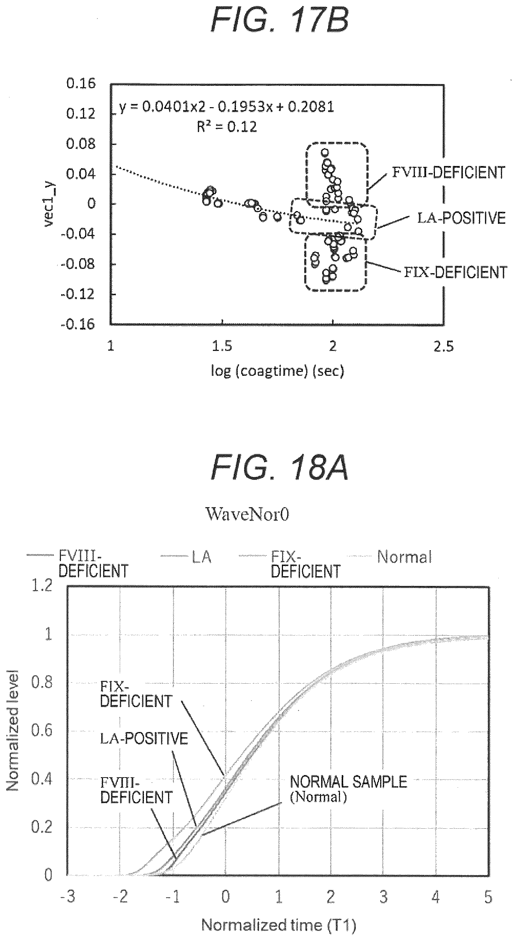

FIG. 17B illustrates an experimental result showing a relationship between vec1_y and a log clotting time in the fourth embodiment. The R2 value obtained by performing least square fitting with a quadratic function was 0.12. That is, it can be seen that vec1_y has almost no correlation with the blood clotting time in the fourth embodiment. A large change amount is shown in the distribution of vec1_y for the FVIII-deficient sample group, the FIX-deficient sample group, and the LA-positive sample group (LA) in FIG. 17B as compared with the total change amount of the index including the normal sample group. Moreover, it can be seen that the sample groups are separated into independent regions in which vec1_y values have a relationship of FVIII-deficient > LA > FIX-deficient.

For a maximum value of a second derivative of a clot waveform obtained by measuring scattered light, which corresponds to another index |min2| described in PTL 1, the result was similar to that of FIG. 17A. As is apparent from the above experimental facts, it is found that a waveform independent of blood clotting time is generated and a feature amount is extracted therefrom by the normalization based on the NRC method according to the present disclosure, thereby improving the performance of identifying the cause of prolongation of the blood clotting time as compared with the conventional index.

FIG. 18A illustrates an experimental result showing a representative waveform of each sample group for WaveNor0 obtained by normalizing a clot waveform of a commercially available sample based on the NRC method in the fourth embodiment. It can be seen that there is a large difference between the sample groups at the rising portion of the waveform.

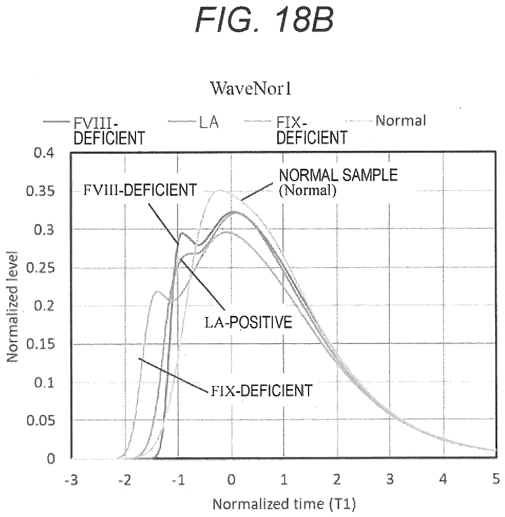

FIG. 18B illustrates an experimental result showing a representative waveform of each sample group for WaveNor1 obtained by normalizing a clot waveform of a commercially available sample based on the NRC method in the fourth embodiment. Similarly, it can be seen that the difference in waveform between the sample groups is clearly shown.

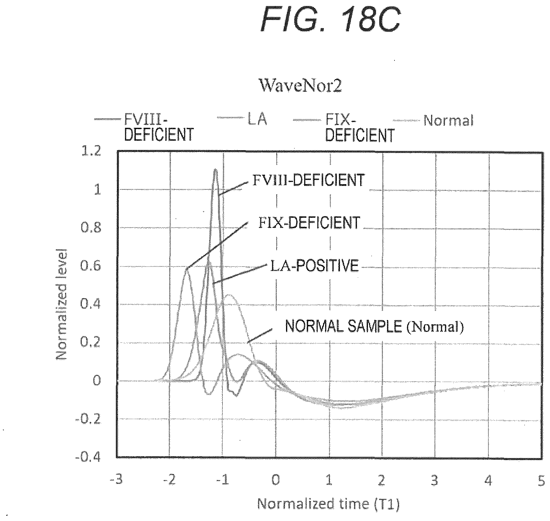

FIG. 18C illustrates an experimental result showing a representative waveform of each sample group for WaveNor2 obtained by normalizing a clot waveform of a commercially available sample based on the NRC method in the fourth embodiment. Similarly, it can be seen that the difference in waveform between the sample groups is clearly shown.

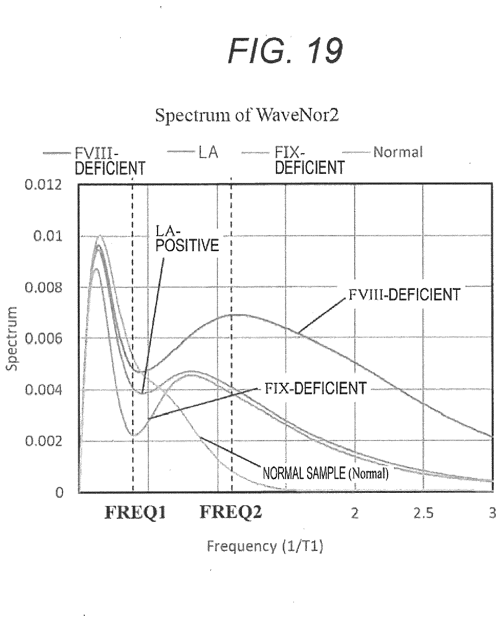

FIG. 19 illustrates an experimental result showing a frequency spectrum of WaveNor2 in the fourth embodiment. Here, for the representative sample of each sample group, a WaveNor2 waveform array of i=0 to 2047 in the normalization time section of -50T1 to +50T1 was generated, and Fourier transform was performed. As the feature of each sample group, for example, amplitude spectrum values of frequencies FREQ1 (minimum value of HB, i=40) and FREQ2 (maximum value of HA, i=115) can be added.

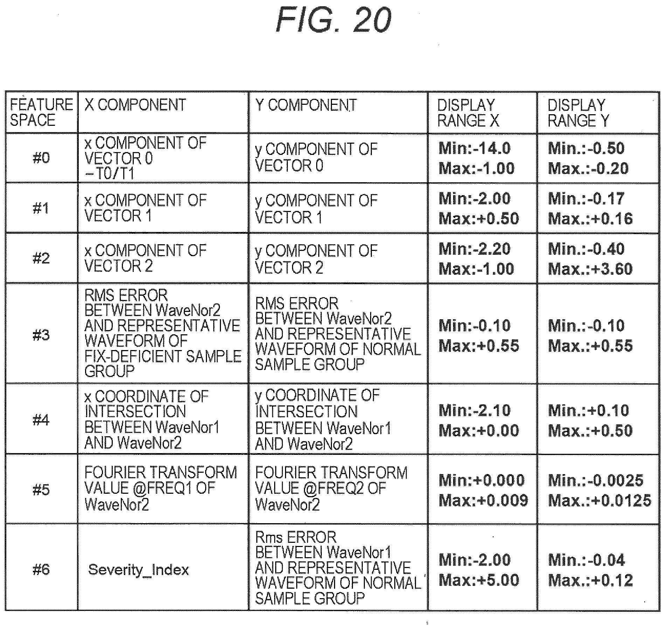

FIG. 20 summarizes an example of a feature set in the fourth embodiment. In general, the more features, the more preferable because it is possible to discriminate the cause of prolongation of the blood clotting time from different perspectives. However, there are limitations thereto. For example, when there are features $\alpha$ and $\beta$, it is not preferable to newly add $\gamma=a\alpha+b\beta$ (a and b are constants) to the features. This is because y is a linear combination of $\alpha$ and $\beta$, and even if $\gamma$ is added to the features, the amount of information for discrimination does not increase, and it is not possible to discriminate of the cause of prolongation of the blood clotting time from a different viewpoint. Furthermore, the addition of y increases the influence of measurement noise and numerical calculation errors, impairing the reliability of the discrimination of the cause of prolongation of the blood clotting time. On the other hand, waveform data obtained by mapping (converting into new information) the normalized waveform (WaveNor0, WaveNorl, WaveNor2) to another space using a nonlinear operation such as a power operation, a differential operation, a Fourier transform operation, a Galois transform operation, or a convolution operation is simple to add an effective feature because its mathematical orthogonality to the original data is guaranteed. Of course, it is also possible to select a feature by listing a plurality of feature candidates from the same waveform data, obtaining cross-correlation coefficients of all combinations thereof, and excluding a combination of feature candidates having a large cross-correlation coefficient (reducing the amount of information for discrimination to be added). Hereinafter, an embodiment in which a value obtained from waveform data mapped to another independent space is added will be described. As illustrated in FIG. 20, here, two features can be used from each of the seven feature spaces (#0 to #6).

(1) Feature space #0
vec0_x-(T0/T1) and vec0_y in the first embodiment
(2) Feature space #1
vec1_x and vec1_y in the first embodiment
(3) Feature space #2
vec2_x and vec2_y in the first embodiment
(4) Feature space #3
RMS error between WaveNor2 and representative waveform of FIX-deficient sample group, and RMS error between WaveNor2 and representative waveform of normal sample group.
(5) Feature space #4
x coordinate and y coordinate of intersection between WaveNor1 and WaveNor2.
(6) Feature space #5
amplitude spectrum value of FREQ1 and amplitude spectrum value of FREQ2 in frequency spectrum of WaveNor2 illustrated in FIG. 19
(7) Feature space #6
numerical value (Severity_Index) defined by the following Mathematical Formula 9, which is experimental formula representing a severity when clotting time is represented by t50, and RMS error between WaveNor1 and representative waveform of normal sample group

Note that an average waveform may be used as the representative waveform described above. The average waveform is a waveform obtained by normalizing waveforms obtained from a group of samples with similar blood coagulation factor activity values based on the NRC method and then averaging the normalization levels for each normalization time.

[Mathematical formula 9]

$$SeverityIndex = 2.41 \log\left(\frac{t50}{28.03}\right)$$

In FIG. 20, x and y ranges recommended when each feature space is illustrated are added for convenience in implementation of numerical analysis. Hereinafter, in the fourth embodiment, the drawings will be disclosed in accordance with these ranges.

In the fourth embodiment, discrimination according to the probability density distribution based on the two-dimensional mixed Gaussian distribution is performed in each feature space as feature discrimination processing. The basic mixed Gaussian distribution will not be described here because it is familiar to a person skilled in the art. In the fourth embodiment, in order to improve the reliability of the discrimination result, the upper limit of the Mahalanobis distance was applied to the probability density distribution of each cluster, on the basis of the mixed Gaussian distribution, in which the influence of measurement errors can be physically inherent in accordance with the central limit theorem. The Mahalanobis distance is an amount equivalent to the standard deviation of the one-dimensional Gaussian distribution, and the upper limit is preferably about 3 to $6\sigma$ when $\sigma$ is designated as a unit according to convention. By applying the upper limit of the Mahalanobis distance, the basic shape of each cluster region on the feature space becomes an ellipse. When two clusters are adjacent to each other, the boundary is a condition that the probability of belonging to each cluster is 50%. Data for one sample is displayed as one point in each feature space, and at the same time, a probability of belonging to each sample group cluster is calculated. The discrimination processing in the fourth embodiment is performed by averaging processing on probabilities of belonging to a cluster in the respective feature spaces, and the cluster in the sample group to which the sample belongs with the highest probability and the belonging probability are obtained as a discrimination result. In the present embodiment, a probability that the sample belongs to each sample group cluster is obtained by averaging processing on probabilities of belonging to a sample group cluster in the respective feature spaces. Alternatively, for example, the probabilities of belonging to a cluster in the respective feature spaces may be weighted-averaged and the sample may be discriminated as a sample group having the highest belonging probability, or may be discriminated from the probability of belonging to the sample group cluster in any one of the feature spaces.

The application of the upper limit of the Mahalanobis distance introduced in the fourth embodiment means that, for example, a data point more than $3\sigma$ away from the center of the sample group cluster does not belong to the cluster in view of the standard deviation of the measurement data. In discrimination using general statistical distribution, SVM, K-means, deep neural network, or the like, the space is divided into a specified number of clusters, so that new or abnormal data is classified into one of the known clusters.

Data for each cluster can be generated by extracting two-feature data from each of the seven feature spaces using clot waveform data from FVIII-deficient samples (FVIII-deficient), FIX-deficient samples (FIX-deficient), LA-positive samples (LA), and normal samples (Normal), and calculating distribution data including a center position, a height, and a variance-covariance matrix to form a cluster for each sample group from the extracted feature data.

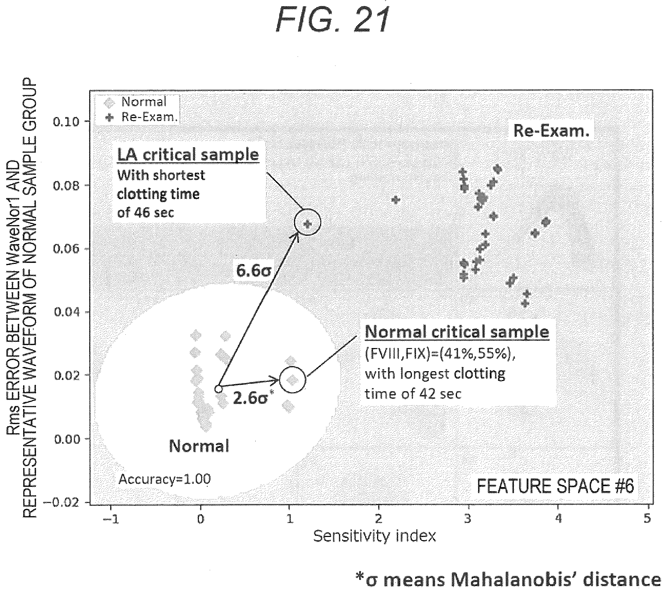

Feature space #6 will be additionally described. FIG. 21 illustrates an experimental result in which a commercially available sample group is displayed on feature space #6 in the fourth embodiment. As illustrated in FIG. 21, this uses Severity_Index defined in Formula 9 as an x-axis feature, and this is specialized for distinguishing between normal samples and other samples, while the other feature spaces distinguish between four sample group clusters. Among the prepared commercially available samples, it has been confirmed that it is possible to separate Borderline Factor Assay Control Plasma (manufactured by George King BioMedical, Inc.) having a clotting time of 42 seconds and an LA-positive sample having a clotting time of 46 seconds at a Mahalanobis distance of $3\sigma$ or more, thereby discriminating them almost perfectly. The FVIII activity value and the FIX activity value of Borderline Factor Assay Control Plasma were 41% and 55%, respectively, according to the attached assay values, and this sample could not be classified into any of the LA-positive sample group, the coagulation factor inhibitor-positive sample group, the FVIII-deficient sample group, and the FIX-deficient sample group. For convenience, this sample was treated as a normal sample in the present embodiment. By changing the upper limit of the Mahalanobis distance, the normal range can be adjusted.

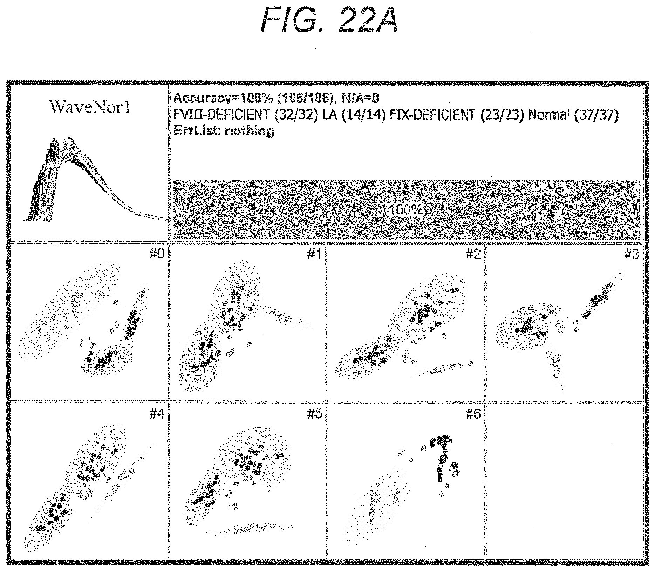

FIG. 22A illustrates an example of a display showing a summary in which commercially available sample groups are plotted on a feature space in the fourth embodiment. In the example of FIG. 22A, while the commercially available sample groups used in the experiment of the fourth embodiment are plotted on seven feature spaces, WaveNor1 obtained by normalization, and a summary of an accuracy rate of a discrimination result are displayed. Here, the upper limit of the Mahalanobis distance was set to $4\sigma$. The accuracy rate was 100% (106/106*100%).

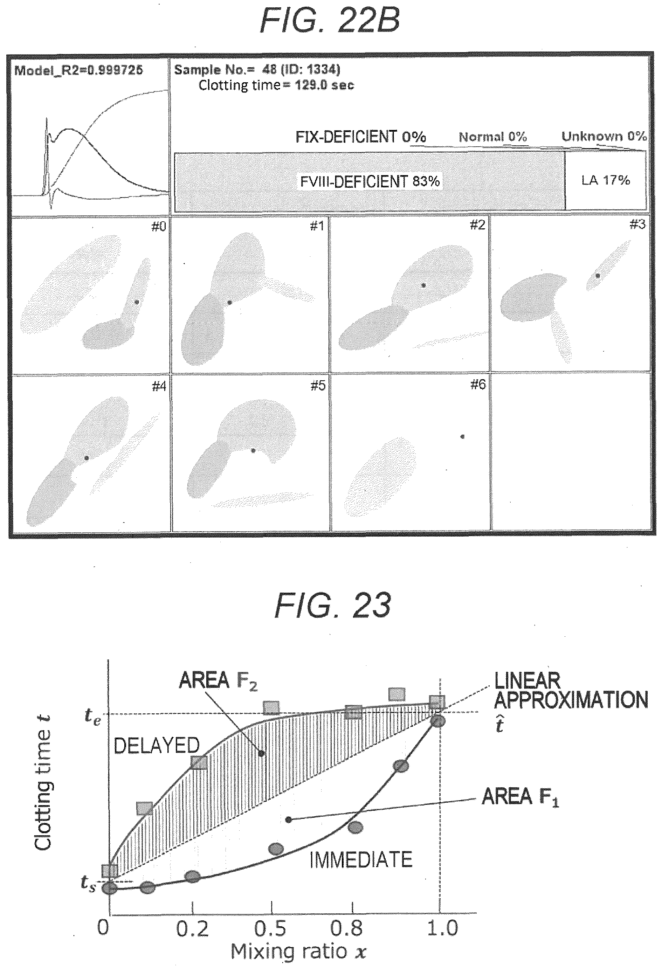

FIG. 22B illustrates an example of a display of a discrimination result of one FVIII-deficient sample in the fourth embodiment. In each of the feature spaces in the fourth embodiment, the sample is expressed as one point, and a probability of belonging to each sample group cluster is presented. In this case, belonging probabilities for FVIII-deficient (83%), LA (17%), FIX-deficient (0%), and Normal (0%) are presented.

[Fifth Embodiment]

In the fifth embodiment, a specific method in a case where the technology of the present invention is applied to the cross mixing test will be described in accordance with the outline presented in the third embodiment. Features are extracted from a first normalized waveform (WaveNorO), a second normalized waveform (WaveNor1), and a third normalized waveform (WaveNor2) for each of a series of samples obtained by mixing plasma separated from blood acquired from a subject and normal plasma at a plurality of mixing ratios, and features are extracted from waveform data obtained by performing a nonlinear operation thereon, and a cause of prolongation of a blood clotting time of the test sample is estimated using at least one of the features.

Regarding the cross mixing test, paragraph 0121 of PTL 1 describes that "Changes in graph patterns of the LA-positive sample and the coagulation factor inhibitor-positive sample were similar. Therefore, the discrimination between the two samples based on the changes in graph patterns must be a qualitative evaluation, and it can be understood that it is difficult for a non-expert to make a determination." As described above, it is a well-known fact that it is difficult to discriminate between the LA-positive sample and the coagulation factor inhibitor-positive sample from the relationship between the mixing ratio between normal plasma and test plasma and the clotting time. An object of the present embodiment is to provide a method capable of not only discriminating between an LA-positive sample group and a coagulation factor inhibitor-positive sample group, but also enabling even a non-expert to quantitatively discriminate a cause of prolongation of a blood clotting time, including a FVIII-deficient sample group and a FIX-deficient sample group, by applying the technology for solving problem (1) to a cross mixing test

The method for estimating a cause of prolongation of a blood clotting time for a test sample in the fifth embodiment includes:

(A) preparing a series of samples by mixing plasma separated from blood acquired from a subject and normal plasma at a plurality of mixing ratios;
(B) acquiring a series of clot waveforms showing changes in light amount over time according to coagulation reactions of reaction liquids generated by mixing the series of samples and a reagent;
(C) acquiring a series of first waveforms by performing before-after differentiation processing on the series of clot waveforms, respectively;
(D) acquiring a series of first fitted waveforms by performing fitting processing on the series of clot waveforms, respectively;
(E) acquiring a series of second fitted waveforms by performing fitting processing on the series of first waveforms, respectively;
(F) acquiring a series of third waveforms by performing before-after differentiation processing on the series of second fitted waveforms, respectively;
(G) acquiring a series of first normalized waveforms, a series of second normalized waveforms, and a series of third normalized waveforms by normalizing the series of first fitted waveforms, the series of second fitted waveforms, and the series of third waveforms, respectively, on a light amount axis and a time axis;
(H) extracting features from at least one of the series of first normalized waveforms, the series of second normalized waveforms, the series of third normalized waveforms, or a series of waveform data obtained by performing a nonlinear operation thereon; and
(I) estimating a cause of prolongation of the blood clotting time for the sample using at least one of the series of features extracted from the series of clot waveforms.

A computer system such as the control computer 120 described in the first embodiment executes each of the steps (A) to (I) described above.

Hereinafter, in the present embodiment, the FVIII-deficient sample group will be referred to as "FVIII-deficient", the FIX-deficient sample group will be referred to as "FIX-deficient", the LA-positive sample group will be referred to as "LA", and the coagulation factor inhibitor-positive sample group (here, commercially available products obtained by adding a coagulation factor inhibitor to the FVIII-deficient sample group) will be referred to as "FVIII-deficient inh", in order to simplify the drawings and help understanding of the technology. For the experiments, commercially available samples were used. The specific samples used are as follows. The FVIII-deficient samples are Factor VIII Deficient Plasma (manufactured by Precision BioLogic, Inc.). The FIX-deficient samples are Factor IX Deficient Plasma (manufactured by Precision BioLogic, Inc.). The LA-positive samples are Lupus Positive Control and Weak Lupus Positive Control (manufactured by Precision BioLogic, Inc.). The coagulation factor inhibitor-positive samples are Mild Factor VIII Inhibitor Plasma and Strong Factor VIII Inhibitor Plasma (manufactured by Affinity Biologicals, Inc.). The normal samples are Pooled Normal Plasma (manufactured by Precision BioLogic, Inc.). Here, the FVIII-deficient samples, the FIX-deficient samples, the LA-positive samples, and the coagulation factor inhibitor-positive samples were used as test samples. In the present embodiment, data from repeated measurements was treated as one sample, a total of 34 test samples (FVIII-deficient: 7, FIX-deficient: 7, LA: 8, FVIII-deficient inh: 12) were considered. The measurement device is Hitachi Automatic Analyzer 3500 (manufactured by Hitachi High-Tech Corporation), and the reagent is Coagpia APTT-N (manufactured by Sekisui Medical Co., Ltd.).

In the present embodiment, a series of plasma samples were prepared by mixing a test sample and a normal sample at different ratios (10:0, 9:1, 8:2, 5:5, 2:8, 1:9, 0:10) were prepared, and clot waveforms were measured immediately after the samples were prepared (immediate-type) and after the samples were heated at 37°C for 2 hours (delayed-type). For one test sample, a series of 14 (7 series of immediate-type and delayed-type) clot waveforms were measured, and features extracted from waveforms normalized based on the NRC method were calculated. Hereinafter, in order to represent the mixed plasma samples, seven mixing ratios (0.0, 0.1, 0.2, 0.5, 0.8, 0.9, and 1.0) and measurement conditions (immediate and delayed) of the test sample are used.

FIG. 23 is a schematic diagram illustrating a method of dimensionally compressing a series of clotting time data obtained for one test sample into two area indexes $F_1$ and $F_2$ in the cross mixing test in the fifth embodiment. In the cross mixing test, there is a condition that a change in blood clotting time is small with respect to a change in test sample mixing ratio. At this time, the obtained blood clotting time data varies greatly as being strongly influenced by mixing conditions and measurement errors. Therefore, an average value of two clotting times (immediate time and delayed time) in a case where the mixing ratio is 0.0 is set as $t_s$, an average value of two clotting times (immediate time and delayed time) in a case where the mixing ratio is 1.0 is set as $t_e$, and the differences from the linearly approximated clotting time are quantified as area indexes $F_1$ and $F_2$ based on the following formula, thereby performing indexing by reducing the variation in measurement result through dimensional compression and averaging. In the following formula, n is an integer (n=1 or 2), N is the number of mixing ratios (here, N=7), i is an index integer representing the mixing ratio, $t_i$ is a clotting time, $\hat{t}_i$ is a clotting time linearly approximated from $t_s$ and $t_e$, $\Delta x_i$ is an integration section corresponding to an i-th mixing ratio $x_i$, and is a width (at the left end of the width, $x_{i-1}$=0.0, and at the right end of the width, $x_{i+1}$=1.0) connecting intermediate points of $x_{i-1}$ and $x_{i+1}$. Under the experimental conditions implemented in the present embodiment, $\Delta x_i$=(0.05, 0.1, 0.2, 0.3, 0.2, 0.1, 0.05) for the mixing ratio $x_i$=(0.0, 0.1, 0.2, 0.5, 0.8, 0.9, 1.0).

[Mathematical formula 10]

$$F_n = 2 \sum_{i=1}^{N} \frac{t_i - \hat{t}_i}{t_e - t_s} \Delta x_i$$

When the index $F_1$ of immediate measurement and the index $F_2$ of delayed measurement are calculated according to the above formula, for example, even if the mixing ratios used in the immediate measurement and the delayed measurement are different, indexes of the same dimension can be obtained as $-1 \leq F_1 \leq +1$ and $-1 \leq F_2 \leq +1$.

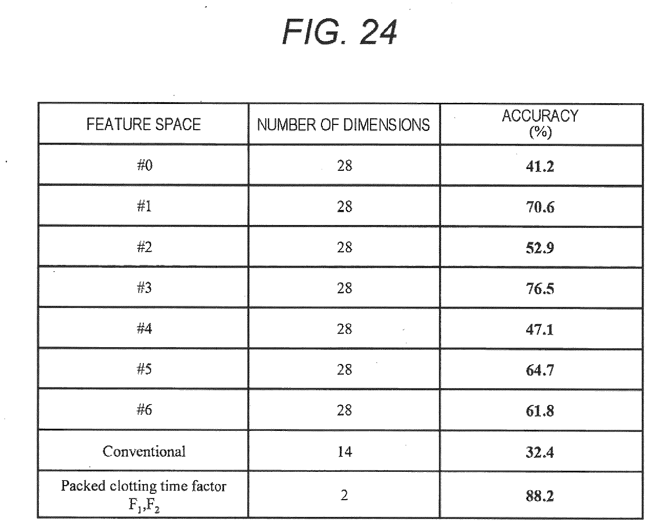

FIG. 24 is a diagram illustrating a discrimination result of samples in a cross mixing test using the multidimensional mixed Gaussian distribution in the fifth embodiment. Here, a case will be described in which a series of 14 test samples are prepared and tested, changing the mixing ratio between one original sample and the normal sample and changing immediate and delayed time conditions. In the method disclosed in the first embodiment, one clot waveform is associated with one original test sample. When this is expanded to a case where 14 clot waveforms are associated with one original test sample, 28 features per feature space are quantified. In each feature space, the discrimination accuracy was quantified by expanding the two-dimensional mixed Gaussian distribution described in the first embodiment to a 28-dimensional mixed Gaussian distribution. Raw data of blood clotting time was discriminated by a 14-dimensional mixed Gaussian distribution as conventional, and the above-described dimensionally compressed index ($F_1$, $F_2$) was discriminated by a two-dimensional mixed Gaussian distribution, and the results were summarized. As can be seen from FIG. 24, the discrimination accuracy was in the range of 32.4% to 88.2%, with the lowest discrimination accuracy when raw data of blood clotting time was used, and the highest discrimination accuracy when the index ($F_1$, $F_2$) was used. The two methods are essentially the same. However, the method using ($F_1$, $F_2$), which reduces the influence of variation, can obtain higher discrimination accuracy as described above. Therefore, in the cross mixing test, only the expansion of the method disclosed in the first embodiment by the conditions for preparing a series of mixed plasma samples and the like is a highly difficult measurement that cannot be handled.

When the indexes of the feature spaces (28 dimensions) obtained by the normalization based on the NRC method of the present embodiment are used, the maximum value 76.5% of the discrimination accuracy is obtained in feature space #3. This is also believed to be strongly influenced by measurement variation.

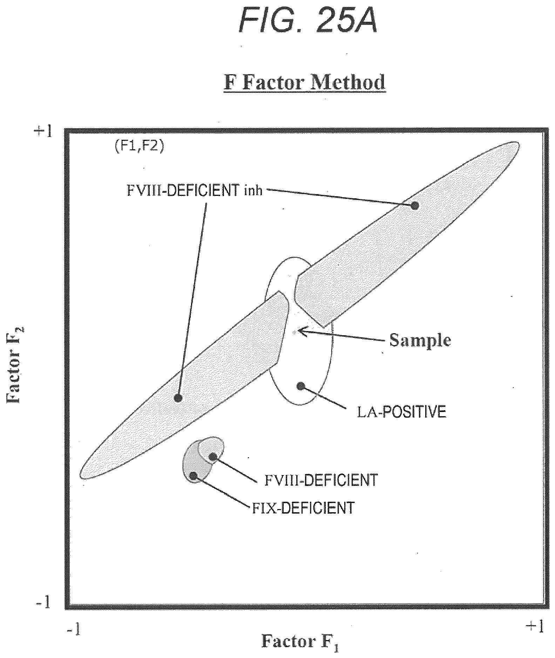

FIG. 25A is a diagram illustrating cluster regions of the indexes $(F_1, F_2)$ in the fifth embodiment. As can be seen from FIG. 25A, the FVIII-deficient cluster and the FIX-deficient cluster are formed close to each other to almost overlap each other. It can also be seen that it is difficult to distinguish the FVIII-deficient cluster and the FIX-deficient cluster from each other with high accuracy from the viewpoint of which of the FVIII factor and the FIX factor is deficient although the FVIII-deficient cluster and the FIX-deficient cluster are independent clusters as blood coagulation factor deficient groups. Although it is difficult to specify which blood coagulation factor is deficient, it can be determined that the cause of prolongation of the blood clotting time is of a factor deficient type through the features of the conventional cross mixing test. The LA-positive cluster (LA) is formed to overlap a central portion of the coagulation factor inhibitor-positive cluster (FVIII-deficient inh), and experimental results that reproduced the description in paragraph 0121 of PTL 1 were obtained: "Changes in graph patterns of the LA-positive sample and the coagulation factor inhibitor-positive sample were similar. Therefore, the discrimination between the two samples based on the changes in graph patterns must be a qualitative evaluation, and it can be understood that it is difficult for a non-expert to make a determination."

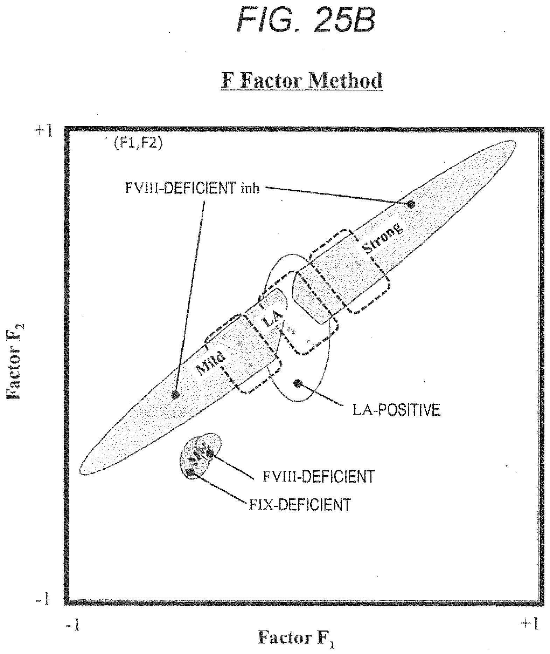

FIG. 25B is a diagram illustrating an experimental result obtained by adding data points to the cluster regions of the indexes $(F_1, F_2)$ in the fifth embodiment. As can be seen from FIG. 25B, it can be understood that, regarding the coagulation factor inhibitor-positive cluster (FVIII-deficient inh), two types of commercially available samples whose inhibitor titers are "Mild" and "Strong" are prepared, and are distributed on the lower left side and the upper right side with the LA-positive cluster (LA) interposed therebetween. Therefore, assuming a sample from a patient at an intermediate level between "Mild" and "Strong", FVIII-deficient inh ≒ LA, and it is considered that it is not possible to discriminate therebetween by this method.

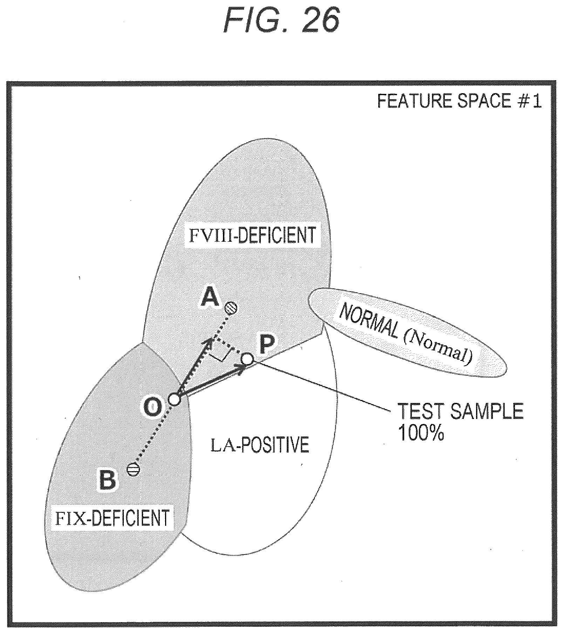

FIG. 26 is a diagram for explaining indexes $(G_1, G_2)$ in the fifth embodiment. As described above, it is difficult to separate the FVIII-deficient cluster and the FIX-deficient cluster only by using the blood clotting time. Therefore, with the midpoint of the line segment connecting the center of the FVIII-deficient cluster and the center of the FIX-deficient cluster in each feature space as the origin, a length of a perpendicular line from the position vector up to the data P of the test sample 100% is used as an index. The indexes $(G_1, G_2)$ corresponding to the immediate measurement and the delayed measurement is defined by the following formula.

[Mathematical formula 11]

$$G_n = \frac{1}{2} \times \frac{vecOP \cdot vecOA}{|vecOA|^2}$$

In the above formula, the center of the FVIII-deficient cluster is +0.5, and the center of the FIX-deficient cluster is -0.5, which means that the sample is more likely to be a FVIII-deficient sample as the numerical value is larger.

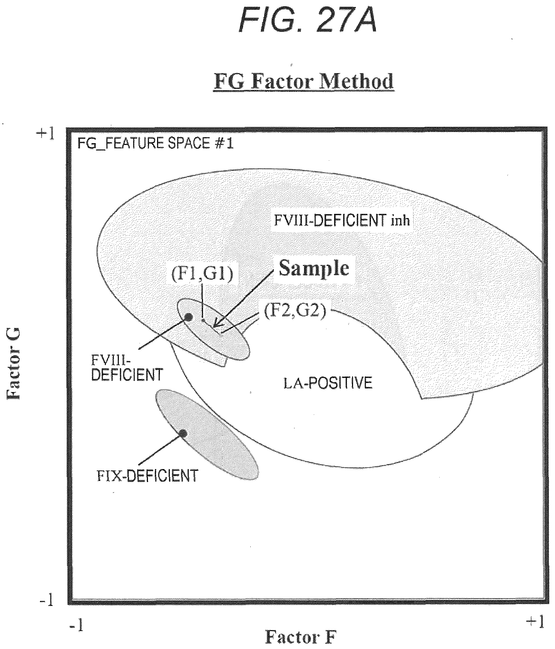

FIG. 27A is a diagram (an example of a display) illustrating an experimental result in which immediate measurement indexes $(F_1, G_1)$ and delayed measurement indexes $(F_2, G_2)$ are plotted, $(F_1, G_1)$ and $(F_2, G_2)$ being obtained by combining an index F calculated from a series of clot waveforms obtained from one sample and an index G calculated from the data in feature space #1 using the same one sample according to Formula 11 in the fifth embodiment. Here, one sample is represented as two points $(F_1, G_1)$ and $(F_2, G_2)$ and a straight line connecting them. The appearance is similar to a schematic diagram of a dipole. Hereinafter, this will be referred to as a dipole. As can be seen from FIG. 27A, the clusters of the four sample groups (FVIII-deficient, FIX-deficient, LA, FVIII-deficient inh) are formed in almost independent regions. The upper limit of the Mahalanobis distance was set to 6σ. The reason why the region of FVIII-deficient inh is relatively wide is that there are two types of inhibitor titers "Mild" and "Strong" as described above.

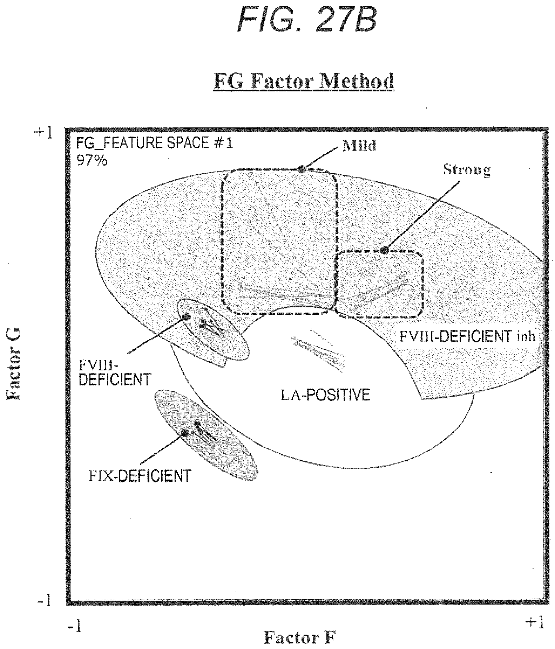

FIG. 27B is a diagram (an example of a display) illustrating an experimental result in which indexes $(F_1, G_1)$ and $(F_2, G_2)$ obtained from 34 samples are plotted in the fifth embodiment. Here, the index G is calculated from data for feature space #1 according to Formula 11. It can be seen that dipoles representing respective samples are separated and plotted according to the causes of prolongation of the blood clotting time. The discrimination accuracy was 97%. It can be seen that "Mild" and "Strong" of the coagulation factor inhibitor-positive group (FVIII-deficient inh), the LA-positive group (LA), the FVIII-deficient group, and the FIX-deficient group are spatially separated and plotted. It was confirmed that the discrimination accuracy was significantly improved by this method as compared with that of the conventional discrimination only using the blood clotting time.

Next, as a specific example of a method suitable for applying the technology of the present invention to the cross mixing test, a method of discriminating a result of a cross mixing test using feature spaces #0 to #5 will be described.

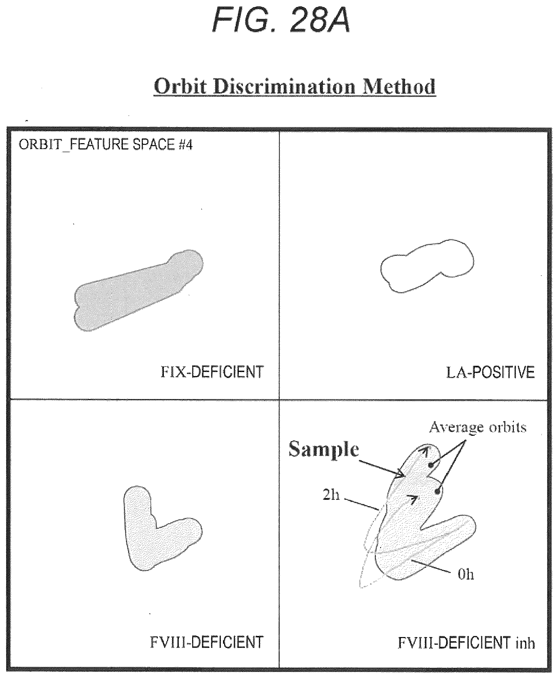

On the feature space, a sequence of seven points obtained by the immediate measurement and the delayed measurement of one sample, which are connected in ascending or descending order of test sample mixing ratio, is referred to as orbit data here. FIG. 28A is an experimental result (an example of a display) showing an average shape of orbit data for each cause of prolongation of the blood clotting time obtained by the cross mixing test in the fifth embodiment. As can be seen from FIG. 28A, the shape of the orbit data is characteristic for each cause of prolongation of the blood clotting time. For example, in terms of morphology (shape similarity), the FVIII-deficient group and the coagulation factor inhibitor-positive group (FVIII-deficient inh) are similar to each other, but a large discrepancy between immediate (Wait=0h) and delayed (Wait=2h) in the coagulation factor inhibitor-positive group (FVIII-deficient inh) can be determined to be a correct result in light of the essence of the cross mixing test.

It is effective to apply a handwritten character recognition technology such as convolutional neural network (CNN) as one of methods for identifying the cause of prolongation of the blood clotting time from the orbit data. This is a method in which points constituting orbit data are connected using straight lines, by spline interpolation, or the like, to generate image data to be discriminated by the same method as handwritten character recognition. This method is capable of discriminating many causes of prolongation of the blood clotting time from each other depending on not only the clusters of the four sample groups handled this time but also conditions of patients under treatment (including medication and the like).

As another method for identifying the cause of prolongation of the blood clotting time from the orbit data, a multidimensional mixed Gaussian distribution can be used. As illustrated in FIG. 24, it is known that the result thereof cannot be obtained with high accuracy. The main reason is that the cluster of each sample group is defined as the inside of a 28-dimensional solid according to the above-described conditions, and it is determined that the mathematical restriction that the solid should be a Gaussian distribution regardless of the plane to be sliced causes an insufficient degree of freedom in adaption to the discrimination of the orbit data of the cross mixing test. As is well known, it must be non-degenerate in a multidimensional mixed Gaussian distribution, whereas the limitations are relaxed in a two-dimensional mixed Gaussian distribution. Therefore, by separating the 28-dimensional orbit data into 2 dimensions x 14 spaces and discriminating the cause of prolongation of the blood clotting time using the product of the probabilities of belonging to the cluster of the sample group obtained in the respective spaces, the cause of prolongation of the blood clotting time can be discriminated, not bound by the restriction of the continuity of the orbit data with respect to the change in sample mixing ratio.

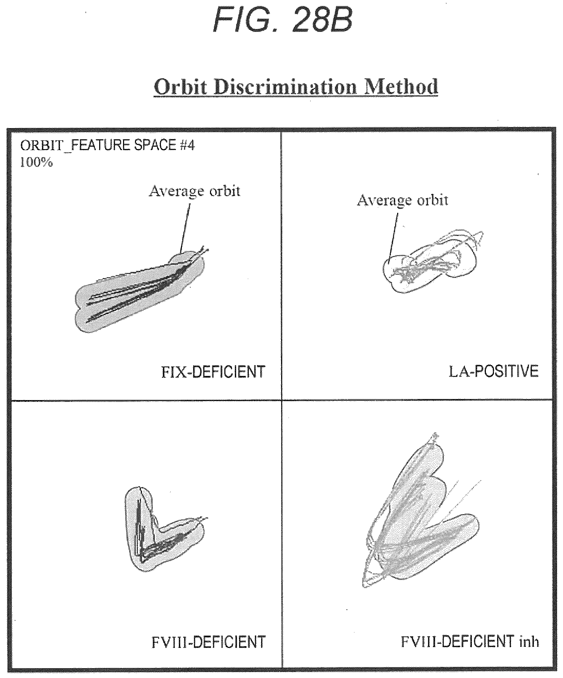

FIG. 28B illustrates an experimental result (an example of a display) obtained by performing discrimination by separating orbit data of each sample in the cross mixing test into 2 dimensions x 14 spaces. Here, the discrimination was performed from the normalized probability of belonging to the cluster of the sample group based on the product of the belonging probabilities obtained in the individual spaces, and orbit data was plotted into four divided regions. In FIG. 28B, the result of feature space #4 is shown as an example. A significant improvement was demonstrated as compared to FIG. 24 (discrimination accuracy =32.4% to 88.2%), which is a result of simple expansion of the method disclosed in the first embodiment, and the discrimination accuracy was 100%.

The complicated sample preparation and incubation procedures involved in the cross mixing test place a burden on an examiner. Additional blood collection for examination also increases the burden on the patient. Therefore, a smaller number of sample conditions (the total number of samples to be prepared depending on differences in mixing ratio and incubation) used in a cross mixing test is more preferable.

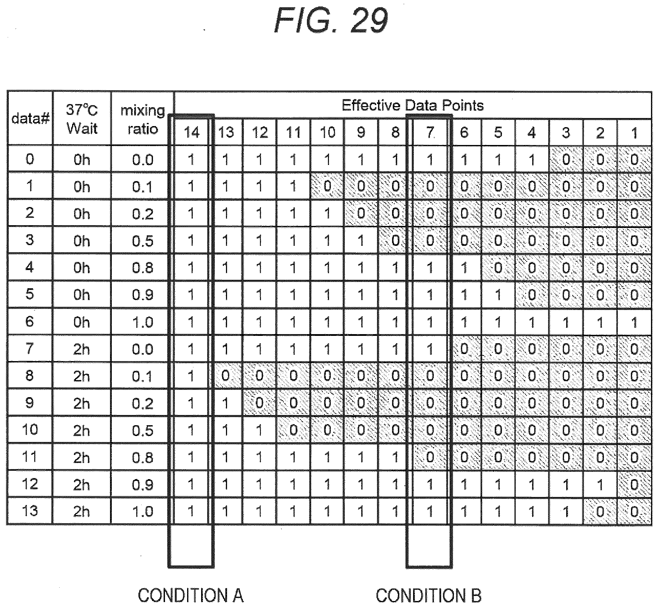

FIG. 29 is a diagram in which conditions for investigating the total number of samples required for the discrimination method are summarized in the fifth embodiment. As described above, this time, 14 (7 series of immediate-type and delayed-type) clot waveforms were acquired for one sample. Each row indicates a clot waveform condition, and hatched condition indicates that it is not used for clot waveform analysis. In FIG. 29, "condition A" indicates a case where all 14 clot waveforms are used to perform discrimination, and "condition B" indicates a case where 7 clot waveforms are used, excluding the hatched waveforms, to perform discrimination.

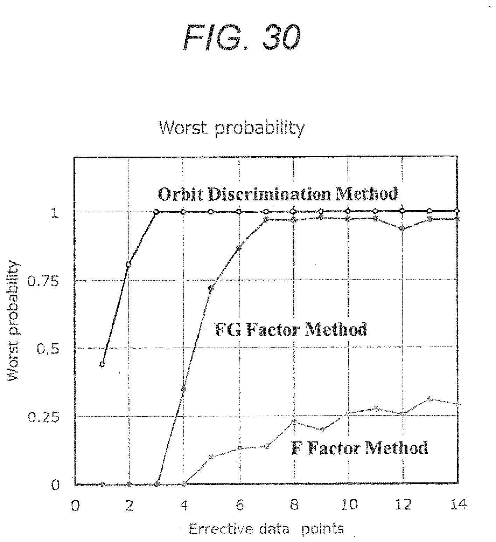

FIG. 30 is a schematic diagram illustrating a relationship between the number of clot waveforms used for discrimination and a worst probability (a minimum value of Pti among all the samples, assuming that Pti is a probability that an i-th sample belongs to the correct sample group cluster) in the fifth embodiment. It can be considered that a discrimination error does not occur when the worst probability on the vertical axis is 50% or more, and a discrimination error occurs when the worst probability on the vertical axis is 50% or less. Here, in terms of performance, the three methods were compared: (1) discrimination using indexes $F_1$ and $F_2$ (F Factor Method), (2) discrimination using a combination of indexes $F_1$, $G_1$, $F_2$, and $G_2$ (FG Factor Method), and (3) discrimination in which orbit data is divided into two-dimensional sets (Orbit Discrimination Method). (1)In the F Factor Method, the worst probability was 50% or less in all conditions, and discrimination errors always occurred. (2)In the FG Factor Method, the discrimination performance was improved, and the worst probability was 50% or more when five or more clot waveforms were used. (3)In the orbit Discrimination Method, the best performance was exhibited, and the worst probability was 77% or more when two or more clot waveforms were used.

The F Factor Method uses a relationship between a condition for mixing a test sample and a normal sample and a blood clotting time that is indexed according to the definition described above. Therefore, it can be considered that the discrimination performance thereof is equivalent to the discrimination result of the conventional cross mixing test.

In the FG Factor Method, shape features of clot waveforms quantified by the NRC method of the present invention are added as indexes $G_1$ and $G_2$ to the indexes $F_1$ and $F_2$, thereby improving the discrimination performance as illustrated in FIG. 30. Since the amount of data to be processed is as small as four pieces per sample, the information processing calculation can be performed in a shorter period of time as compared with that in the Orbit Discrimination Method. Therefore, the FG Factor Method is suitable for use as auxiliary information when a discrimination result is determined together with the conventional method in which the cause of prolongation of the blood clotting time is discriminated from the relationship between the sample mixing ratio and the blood clotting time.

In the Orbit Discrimination Method, a result of a cross mixing test is discriminated only by an index of a feature space normalized and quantified by the NRC method of the present invention, and high discrimination performance can be obtained. At the same time, since there is no calculation of the index F according to Formula 10, it is possible to perform information calculation independent from the relation with the adjacent data points via $\Delta x_i$, and it is possible to flexibly cope with selection of any mixing ratio. Because of these advantages, the Orbit Discrimination Method is suitable as a means for realizing fully automatic discrimination in a cross mixing test using a machine learning technology.

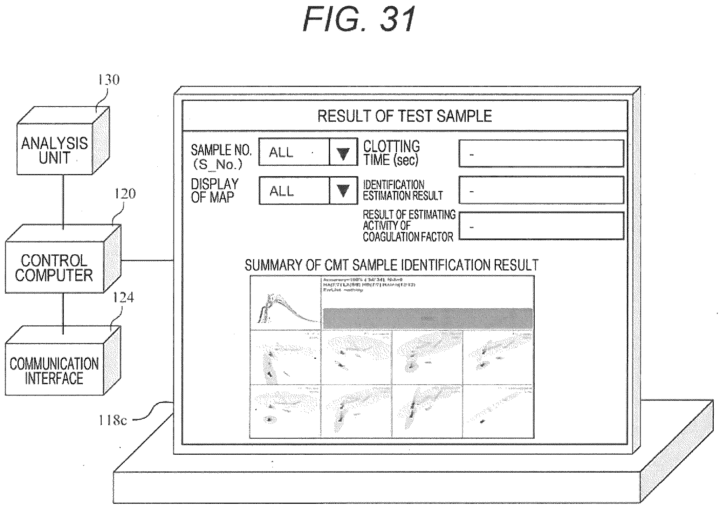

FIG. 31 illustrates an example of a display showing a discrimination result of a cross mixing test in the fifth embodiment. The relationship between the analysis unit 130, the control computer 120, and the communication interface 124 and the display of the result of the test sample on the display unit 118c follow the result in FIG. 9.

According to the fifth embodiment, a series of clot waveforms obtained by a cross mixing test enables quantitative discrimination based on the probabilities for the LA-positive sample group, the coagulation factor inhibitor-positive sample group, the FVIII-deficient sample group, and the FIX-deficient sample group.

[Modification]

It should be noted that the present disclosure is not limited to the above-described embodiments, and includes various modifications. The above-described embodiments have been described in detail in order to explain the present disclosure in an easy-to-understand manner, and are not necessarily limited to having all the configurations described above. A part of the configuration of one embodiment may be replaced with the configuration of another embodiment, and the configuration of one embodiment may be added to the configuration of another embodiment. With respect to a part of the configuration of each embodiment, it is possible to add, delete, or replace the same configuration or another configuration

For example, in the first embodiment, the cause of prolongation of the blood clotting time is estimated from the features of WaveNor0, WaveNor1, and WaveNor2. Alternatively, according to the present disclosure, the cause of prolongation of blood clotting time may be estimated from at least two features of WaveNor0, WaveNor1, and WaveNor2. For example, the cause of prolongation may be estimated from the features of the x component and the y component of WaveNor0, or the cause of prolongation may be estimated from the features of the x component of WaveNor0 and the y component of WaveNor2.

Reference Signs List

| | |
|---|---|
| 100 | automatic analyzer |
| 101 | sample dispensing unit |
| 102 | sample disk |
| 103 | sample container |
| 103a | sample |
| 104 | reaction vessel |
| 105 | sample syringe pump |
| 106 | reagent dispensing unit |
| 107 | reagent disk |
| 108 | reagent container |
| 108a | reagent |
| 109 | reagent heating unit |
| 110 | reagent syringe pump |
| 111 | reaction vessel stock unit |
| 112 | reaction vessel transport unit |

| 113 | detection unit |
| 114 | reaction vessel installation unit |
| 115 | light source |
| 116 | detection unit (optical sensor) |
| 117 | reaction vessel discard unit |
| 118 | operation computer |
| 118a | mouse |
| 118b | keyboard |
| 118c | display unit |
| 119 | storage unit |
| 120 | control computer |
| 121 | A/D converter |
| 122 | incubator |
| 123 | printer |
| 124 | communication interface |
| 125 | analysis computer |
| 130 | analysis unit |

**Claims**

1. A method for estimating a cause of prolongation of a blood clotting time, the method comprising:

   acquiring a clot waveform showing a change in light amount over time according to a coagulation reaction of a reaction liquid generated by mixing a test sample composed of plasma separated from blood acquired from a subject and a reagent;
   acquiring a first waveform by performing before-after differentiation processing on the clot waveform;
   acquiring a first fitted waveform by performing fitting processing on the clot waveform;
   acquiring a second fitted waveform by performing fitting processing on the first waveform;
   acquiring a third waveform by performing before-after differentiation processing on the second fitted waveform;
   acquiring a first normalized waveform, a second normalized waveform, and a third normalized waveform by normalizing the first fitted waveform, the second fitted waveform, and the third waveform, respectively, on a light amount axis and a time axis;
   extracting a feature from each of the first normalized waveform, the second normalized waveform, and the third normalized waveform; and
   estimating a cause of prolongation of a blood clotting time for the test sample based on a known feature extracted from a sample group for which a cause of prolongation of a blood clotting time is known and the feature extracted from each of the first normalized waveform, the second normalized waveform, and the third normalized waveform.

2. The method according to claim 1, wherein
   the normalization is based on a numerical value obtained by performing fitting processing on the clot waveform.

3. The method according to claim 1, wherein
   the estimating of the cause of prolongation of the blood clotting time includes estimating that the cause of prolongation of the blood clotting time for the test sample is blood coagulation factor VIII deficiency, blood coagulation factor IX deficiency, or lupus anticoagulant (LA) positive.

4. The method according to claim 3, wherein
   the blood coagulation factor VIII deficiency is a state in which an activity value of a blood coagulation factor VIII is less than 1%, and the blood coagulation factor IX deficiency is a state in which an activity value of a blood coagulation factor IX is less than 1%.

5. The method according to claim 1, further comprising:

   performing first filter processing for removing noise from the clot waveform before the fitting processing on the clot waveform; and
   performing second filter processing for removing noise from the first waveform before the fitting processing on the first waveform.

6. The method according to claim 5, wherein
in the second filter processing, moving average processing is performed using a reaction time constant that is one of parameters obtained by the fitting processing on the clot waveform.

7. The method according to claim 1, further comprising:
displaying the estimated cause of prolongation on a display.

8. The method according to claim 1, further comprising:

creating a map showing a relationship between the known feature extracted from the sample group for which the cause of prolongation of the blood clotting time is known and the cause of prolongation of the blood clotting time; and
displaying, on a display, the map and information obtained by plotting the feature of the test sample on the map.

9. The method according to claim 1, further comprising:
displaying an identification estimation result based on the estimated cause of prolongation of the blood clotting time for the test sample on a display.

10. An information processing device communicable with an automatic analyzer configured to acquire a clot waveform showing a change in light amount over time according to a coagulation reaction of a reaction liquid generated by mixing a test sample composed of plasma separated from blood acquired from a subject and a reagent, the information processing device comprising:

a computer system including a processor and a memory,
wherein the computer system is configured to execute:

acquiring the clot waveform from the automatic analyzer;
acquiring a first waveform by performing before-after differentiation processing on the clot waveform;
acquiring a first fitted waveform by performing fitting processing on the clot waveform,
acquiring a second fitted waveform by performing fitting processing on the first waveform;
acquiring a third waveform by performing before-after differentiation processing on the second fitted waveform;
acquiring a first normalized waveform, a second normalized waveform, and a third normalized waveform by normalizing the first fitted waveform, the second fitted waveform, and the third waveform, respectively, on a light amount axis and a time axis;
extracting a feature from each of the first normalized waveform, the second normalized waveform, and the third normalized waveform; and
estimating a cause of prolongation of a blood clotting time for the test sample based on a known feature extracted from a sample group for which a cause of prolongation of a blood clotting time is known and the feature extracted from each of the first normalized waveform, the second normalized waveform, and the third normalized waveform.

11. The information processing device according to claim 10, wherein
the computer system is configured to execute normalization based on a numerical value obtained by performing fitting processing on the clot waveform.

12. The information processing device according to claim 10, wherein
the estimating of the cause of prolongation of the blood clotting time includes estimating that the cause of prolongation of the blood clotting time for the test sample is blood coagulation factor VIII deficiency, blood coagulation factor IX deficiency, or lupus anticoagulant (LA) positive.

13. The information processing device according to claim 12, wherein
the blood coagulation factor VIII deficiency is a state in which an activity value of a blood coagulation factor VIII is less than 1%, and the blood coagulation factor IX deficiency is a state in which an activity value of a blood coagulation factor IX is less than 1%.

14. The information processing device according to claim 10, wherein
the computer system is configured to further execute:

performing first filter processing for removing noise from the clot waveform before the fitting processing on the clot waveform; and
performing second filter processing for removing noise from the first waveform before the fitting processing on the first waveform.

15. The information processing device according to claim 14, wherein
in the second filter processing, the computer system is configured to perform moving average processing using a reaction time constant that is one of parameters obtained by the fitting processing on the clot waveform.

16. The information processing device according to claim 10, further comprising:
a display configured to display the estimated cause of prolongation.

17. The information processing device according to claim 10, wherein
the computer system is configured to further execute:

creating a map showing a relationship between the known feature extracted from the sample group for which the cause of prolongation of the blood clotting time is known and the cause of prolongation of the blood clotting time; and
displaying, on a display, the map and information obtained by plotting the feature of the test sample on the map.

18. The information processing device according to claim 10, further comprising:
a display configured to display an identification estimation result based on the estimated cause of prolongation of the blood clotting time for the test sample.

19. A method for estimating a cause of prolongation of a blood clotting time for a test sample from a clot waveform showing a change in light amount over time according to a coagulation reaction of a reaction liquid generated by mixing the test sample composed of plasma separated from blood acquired from a subject and a reagent, the method comprising:

aligning data lengths of clot waveform data;
estimating a plurality of causes of prolongation of blood clotting times based on the clot waveform data, of which the data lengths are aligned, by using a neural network; and
presenting a cause of prolongation of a blood clotting time for the test sample based on probabilities belonging to the estimated causes of prolongation of the blood clotting times.

20. The method according to claim 19, wherein
the neural network is a neural network having 16 or more nodes and 3 or more hidden layers.

21. A method for estimating a cause of prolongation of a blood clotting time, the method comprising:

acquiring a clot waveform showing a change in light amount over time according to a coagulation reaction of a reaction liquid generated by mixing a test sample composed of plasma separated from blood acquired from a subject and a reagent;
acquiring a first waveform by performing before-after differentiation processing on the clot waveform;
acquiring a first fitted waveform by performing fitting processing on the clot waveform;
acquiring a second fitted waveform by performing fitting processing on the first waveform;
acquiring a third waveform by performing before-after differentiation processing on the second fitted waveform;
acquiring a first normalized waveform, a second normalized waveform, and a third normalized waveform by normalizing the first fitted waveform, the second fitted waveform, and the third waveform, respectively, on a light amount axis and a time axis;
extracting a feature from at least one of the first normalized waveform, the second normalized waveform, the third normalized waveform, or waveform data obtained by performing a nonlinear operation thereon; and
estimating a cause of prolongation of a blood clotting time for the test sample based on information on a distribution of features for each cause of prolongation of the blood clotting time prepared in advance and the feature extracted from the clot waveform of the test sample.

22. A method for estimating a cause of prolongation of a blood clotting time, the method comprising:

preparing a series of samples by mixing plasma separated from blood acquired from a subject and normal plasma at a plurality of mixing ratios;

acquiring a series of clot waveforms showing changes in light amount over time according to coagulation reactions of reaction liquids generated by mixing the series of samples and a reagent;

acquiring a series of first waveforms by performing before-after differentiation processing on the series of clot waveforms, respectively;

acquiring a series of first fitted waveforms by performing fitting processing on the series of clot waveforms, respectively;

acquiring a series of second fitted waveforms by performing fitting processing on the series of first waveforms, respectively;

acquiring a series of third waveforms by performing before-after differentiation processing on the series of second fitted waveforms, respectively;

acquiring a series of first normalized waveforms, a series of second normalized waveforms, and a series of third normalized waveforms by normalizing the series of first fitted waveforms, the series of second fitted waveforms, and the series of third waveforms, respectively, on a light amount axis and a time axis;

extracting features from at least one of the series of first normalized waveforms, the series of second normalized waveforms, the series of third normalized waveforms, or a series of waveform data obtained by performing a nonlinear operation thereon; and

estimating a cause of prolongation of a blood clotting time for the sample using at least one of the series of features extracted from the series of clot waveforms.

23. The method according to claim 22, wherein
the features extracted from the series of waveform data include:

a combination $(F_1, G_1)$ of an index $G_1$ calculated from a position where a feature including a plurality of components acquired from a clot waveform of immediate measurement of a sample in which a mixing ratio of the plasma separated from the blood acquired from the subject is 100%, among the series of samples, is plotted in a feature space indicating the plurality of components, and an index $F_1$ calculated from a series of clotting times of immediate measurement of the series of samples; and

a combination $(F_2, G_2)$ of an index $G_2$ calculated from a position where a feature including a plurality of components acquired from a clot waveform of delayed measurement of the sample in which the mixing ratio of the plasma is 100% is plotted in the feature space, and an index $F_2$ calculated from a series of clotting times of delayed measurement of the series of samples, and

the cause of prolongation of the blood clotting time for the sample is estimated using the combination $(F_1, G_1)$ and the combination $(F_2, G_2)$.

24. The method according to claim 22, wherein
the features extracted from the series of waveform data include:

first orbit data obtained by plotting a series of features including a plurality of components acquired from a series of clot waveforms of immediate measurement of the series of samples in a feature space indicating the plurality of components, and connecting the series of features in the feature space in ascending or descending order of plasma mixing ratio; and

second orbit data obtained by plotting a series of features including a plurality of components acquired from a series of clot waveforms of delayed measurement of the series of samples in a feature space, and connecting the series of features in the feature space in ascending or descending order of plasma mixing ratio, and

the cause of prolongation of the blood clotting time for the sample is estimated using the first orbit data and the second orbit data.

25. An information processing device communicable with an automatic analyzer configured to acquire a clot waveform showing a change in light amount over time according to a coagulation reaction of a reaction liquid generated by mixing a test sample composed of plasma separated from blood acquired from a subject and a reagent, the information processing device comprising:

a computer system including a processor and a memory,
wherein the computer system is configured to execute:

acquiring the clot waveform from the automatic analyzer;
acquiring a first waveform by performing before-after differentiation processing on the clot waveform;

acquiring a first fitted waveform by performing fitting processing on the clot waveform,

acquiring a second fitted waveform by performing fitting processing on the first waveform;

acquiring a third waveform by performing before-after differentiation processing on the second fitted waveform;

acquiring a first normalized waveform, a second normalized waveform, and a third normalized waveform by normalizing the first fitted waveform, the second fitted waveform, and the third waveform, respectively, on a light amount axis and a time axis;

extracting a feature from at least one of the first normalized waveform, the second normalized waveform, the third normalized waveform, or waveform data obtained by performing a nonlinear operation thereon; and

estimating a cause of prolongation of a blood clotting time for the test sample based on information on a distribution of features for each cause of prolongation of the blood clotting time prepared in advance and the feature extracted from the clot waveform of the test sample.

26. The information processing device according to claim 25, wherein the computer system is configured to further execute:

creating a graph showing a relationship between the information on the distribution of the features for each cause of prolongation of the blood clotting time prepared in advance and the feature extracted from the clot waveform of the test sample; and

displaying the graph and an estimation result of the cause of prolongation of the blood clotting time for the test sample on a display.

27. An information processing device communicable with an automatic analyzer configured to prepare a series of samples by mixing plasma separated from blood acquired from a subject and normal plasma at a plurality of mixing ratios, and acquire a series of clot waveforms showing changes in light amount over time according to coagulation reactions of reaction liquids generated by mixing the series of samples and a reagent, the information processing device comprising:

a computer system including a processor and a memory,

wherein the computer system is configured to execute:

acquiring the series of clot waveforms from the automatic analyzer;

acquiring a series of first waveforms by performing before-after differentiation processing on the series of clot waveforms, respectively;

acquiring a series of first fitted waveforms by performing fitting processing on the series of clot waveforms, respectively;

acquiring a series of second fitted waveforms by performing fitting processing on the series of first waveforms, respectively;

acquiring a series of third waveforms by performing before-after differentiation processing on the series of second fitted waveforms, respectively;

acquiring a series of first normalized waveforms, a series of second normalized waveforms, and a series of third normalized waveforms by normalizing the series of first fitted waveforms, the series of second fitted waveforms, and the series of third waveforms, respectively, on a light amount axis and a time axis;

extracting features from at least one of the series of first normalized waveforms, the series of second normalized waveforms, the series of third normalized waveforms, or a series of waveform data obtained by performing a nonlinear operation thereon; and

estimating a cause of prolongation of a blood clotting time for the sample using at least one of the series of features extracted from the series of clot waveforms.

28. The information processing device according to claim 27, wherein the features extracted from the series of waveform data include:

a combination $(F_1, G_1)$ of an index $G_1$ calculated from a position where a feature including a plurality of components acquired from a clot waveform of immediate measurement of a sample in which a mixing ratio of the plasma separated from the blood acquired from the subject is 100%, among the series of samples, is plotted in a feature space indicating the plurality of components, and an index $F_1$ calculated from a series of clotting times of immediate measurement of the series of samples; and

a combination $(F_2, G_2)$ of an index $G_2$ calculated from a position where a feature including a plurality of components acquired from a clot waveform of delayed measurement of the sample in which the mixing ratio of the plasma is 100% is plotted in the feature space, and an index $F_2$ calculated from a series of clotting times of delayed measurement of the series of samples,

the cause of prolongation of the blood clotting time for the sample is estimated using the combination $(F_1, G_1)$ and the combination $(F_2, G_2)$, and

the computer system is configured to further execute:

creating a graph showing a relationship between the index $G_1$ and the index $F_1$ and a relationship between the index $G_2$ and the index $F_2$; and

displaying the graph and an estimation result of the cause of prolongation of the blood clotting time for the sample on a display.

29. The information processing device according to claim 27, wherein
the features extracted from the series of waveform data include:

first orbit data obtained by plotting a series of features including a plurality of components acquired from a series of clot waveforms of immediate measurement of the series of samples in a feature space indicating the plurality of components, and connecting the series of features in the feature space in ascending or descending order of plasma mixing ratio; and

second orbit data obtained by plotting a series of features including a plurality of components acquired from a series of clot waveforms of delayed measurement of the series of samples in a feature space, and connecting the series of features in the feature space in ascending or descending order of plasma mixing ratio,

the cause of prolongation of the blood clotting time for the sample is estimated using the first orbit data and the second orbit data, and

the computer system is configured to further execute:

creating a graph showing the first orbit data and the second orbit data; and

displaying the graph and an estimation result of the cause of prolongation of the blood clotting time for the sample on a display.

# FIG. 1

# FIG. 2

# FIG. 3A

MEASURE COAGULATION REACTION

| READ CLOT WAVEFORM DATA | S301a |

| PERFORM PREPROCESSING | S302a |

| PERFORM NORMALIZATION PROCESSING<br>(CORRECTION PROCESSING)<br>(ONLY ON LIGHT AMOUNT AXIS) | S303a |

| CALCULATE WAVEFORM RELATED<br>TO COAGULATION RATE<br>OR COAGULATION ACCELERATION | S304a |

| EXTRACT WAVEFORM PARAMETER<br>(FEATURE) | S305a |

ESTIMATE IDENTIFICATION

# FIG. 3B

MEASURE COAGULATION REACTION

| |
|---|
| READ CLOT WAVEFORM (WaveSrc0) DATA — S301b |
| GENERATE WaveSrc1 — S302b |
| FIT WaveSrc0 (GENERATE Wave0) — S303b |
| FIT WaveSrc1 (GENERATE Wave1) — S304b |
| GENERATE Wave2 — S305b |
| PERFORM NORMALIZATION PROCESSING ON Wave0, Wave1, and Wave2 BASED ON NRC METHOD (ON BOTH TIME AXIS AND LIGHT AMOUNT AXIS) (GENERATE WaveNor0, WaveNor1 AND WaveNor2) — S306b |
| EXTRACT FEATURE — S307b |

ESTIMATE IDENTIFICATION

# FIG. 4

# FIG. 5

# FIG. 6

EP 4 564 007 A1

# FIG. 7

# FIG. 8

Normalized wave series of sample No.8 (S_No=1335)

## FIG. 9

## FIG. 10

## FIG. 11A

## FIG. 11B

## FIG. 11C

# FIG. 12

Model: "sequential"

| Layer (type) | Output Shape | Param # |
|---|---|---|
| layer0_dense (Dense) | (None, 512) | 151552 |
| layer0_dropout (Dropout) | (None, 512) | 0 |
| layer1_dense (Dense) | (None, 512 | 262656 |
| layer1_dropout (Dropout) | (None, 512) | 0 |
| layer2_dense (Dense) | (None, 512) | 262656 |
| layer2_dropout (Dropout) | (None, 512) | 0 |
| layer3_dense (Dense) | (None, 512) | 262656 |
| layer3_dropout (Dropout) | (None, 512) | 0 |
| layer4_dense (Dense) | (None, 4) | 2052 |

Total params: 941,572
Trainable params: 941,572
Non-trainable params: 0

# FIG. 13A

Zero padding

## FIG. 13B

Expansion using the fitting model

## FIG. 13C

Simple expansion

## FIG. 14A

accuracy_test

## FIG. 14B

accuracy_test

## FIG. 15

● loss_study
△ loss_validation
○ loss_test

# FIG. 16

WaveSrc0

# FIG. 17A

## FIG. 17B

y = 0.0401x2 - 0.1953x + 0.2081
R² = 0.12

FVIII-DEFICIENT

LA-POSITIVE

FIX-DEFICIENT

log (coagtime) (sec)

## FIG. 18A

WaveNor0

FVIII-DEFICIENT   LA   FIX-DEFICIENT   Normal

FIX-DEFICIENT

LA-POSITIVE

FVIII-DEFICIENT

NORMAL SAMPLE (Normal)

Normalized time (T1)

# FIG. 18B

WaveNor1

# FIG. 18C

WaveNor2

## FIG. 19

Spectrum of WaveNor2

# FIG. 20

| FEATURE SPACE | X COMPONENT | Y COMPONENT | DISPLAY RANGE X | DISPLAY RANGE Y |
|---|---|---|---|---|
| #0 | x COMPONENT OF VECTOR 0 −T0/T1 | y COMPONENT OF VECTOR 0 | Min.:-14.0 Max:-1.00 | Min.:-0.50 Max.:-0.20 |
| #1 | x COMPONENT OF VECTOR 1 | y COMPONENT OF VECTOR 1 | Min.:-2.00 Max:+0.50 | Min.:-0.17 Max.:+0.16 |
| #2 | x COMPONENT OF VECTOR 2 | y COMPONENT OF VECTOR 2 | Min.:-2.20 Max:-1.00 | Min.:-0.40 Max.:+3.60 |
| #3 | RMS ERROR BETWEEN WaveNor2 AND REPRESENTATIVE WAVEFORM OF FIX-DEFICIENT SAMPLE GROUP | RMS ERROR BETWEEN WaveNor2 AND REPRESENTATIVE WAVEFORM OF NORMAL SAMPLE GROUP | Min.:-0.10 Max:+0.55 | Min.:-0.10 Max.:+0.55 |
| #4 | x COORDINATE OF INTERSECTION BETWEEN WaveNor1 AND WaveNor2 | y COORDINATE OF INTERSECTION BETWEEN WaveNor1 AND WaveNor2 | Min.:-2.10 Max:+0.00 | Min.:+0.10 Max.:+0.50 |
| #5 | FOURIER TRANSFORM VALUE @FREQ1 OF WaveNor2 | FOURIER TRANSFORM VALUE @FREQ2 OF WaveNor2 | Min:+0.000 Max:+0.009 | Min.:-0.0025 Max.:+0.0125 |
| #6 | Severity_Index | Rms ERROR BETWEEN WaveNor1 AND REPRESENTATIVE WAVEFORM OF NORMAL SAMPLE GROUP | Min:-2.00 Max:+5.00 | Min.:-0.04 Max.:+0.12 |

# FIG. 21

*σ means Mahalanobis' distance

# FIG. 22A

## FIG. 22B

## FIG. 23

# FIG. 24

| FEATURE SPACE | NUMBER OF DIMENSIONS | ACCURACY (%) |
|---|---|---|
| #0 | 28 | 41.2 |
| #1 | 28 | 70.6 |
| #2 | 28 | 52.9 |
| #3 | 28 | 76.5 |
| #4 | 28 | 47.1 |
| #5 | 28 | 64.7 |
| #6 | 28 | 61.8 |
| Conventional | 14 | 32.4 |
| Packed clotting time factor $F_1, F_2$ | 2 | 88.2 |

# FIG. 25A

## F Factor Method

# FIG. 25B

## F Factor Method

## FIG. 26

FEATURE SPACE #1

FVIII-DEFICIENT

NORMAL (Normal)

TEST SAMPLE 100%

LA-POSITIVE

FIX-DEFICIENT

# FIG. 27A

## FG Factor Method

# FIG. 27B

## FG Factor Method

# FIG. 28A

## Orbit Discrimination Method

# *FIG. 28B*

## Orbit Discrimination Method

ORBIT_FEATURE SPACE #4
100%

Average orbit

FIX-DEFICIENT

Average orbit

LA-POSITIVE

FVIII-DEFICIENT

FVIII-DEFICIENT inh

# FIG. 29

| data# | 37°C Wait | mixing ratio | Effective Data Points | | | | | | | | | | | | | |
|---|---|---|---|---|---|---|---|---|---|---|---|---|---|---|---|---|
| | | | 14 | 13 | 12 | 11 | 10 | 9 | 8 | 7 | 6 | 5 | 4 | 3 | 2 | 1 |
| 0 | 0h | 0.0 | 1 | 1 | 1 | 1 | 1 | 1 | 1 | 1 | 1 | 1 | 1 | 0 | 0 | 0 |
| 1 | 0h | 0.1 | 1 | 1 | 1 | 1 | 0 | 0 | 0 | 0 | 0 | 0 | 0 | 0 | 0 | 0 |
| 2 | 0h | 0.2 | 1 | 1 | 1 | 1 | 1 | 0 | 0 | 0 | 0 | 0 | 0 | 0 | 0 | 0 |
| 3 | 0h | 0.5 | 1 | 1 | 1 | 1 | 1 | 1 | 0 | 0 | 0 | 0 | 0 | 0 | 0 | 0 |
| 4 | 0h | 0.8 | 1 | 1 | 1 | 1 | 1 | 1 | 1 | 1 | 1 | 0 | 0 | 0 | 0 | 0 |
| 5 | 0h | 0.9 | 1 | 1 | 1 | 1 | 1 | 1 | 1 | 1 | 1 | 1 | 0 | 0 | 0 | 0 |
| 6 | 0h | 1.0 | 1 | 1 | 1 | 1 | 1 | 1 | 1 | 1 | 1 | 1 | 1 | 1 | 1 | 1 |
| 7 | 2h | 0.0 | 1 | 1 | 1 | 1 | 1 | 1 | 1 | 1 | 0 | 0 | 0 | 0 | 0 | 0 |
| 8 | 2h | 0.1 | 1 | 0 | 0 | 0 | 0 | 0 | 0 | 0 | 0 | 0 | 0 | 0 | 0 | 0 |
| 9 | 2h | 0.2 | 1 | 1 | 0 | 0 | 0 | 0 | 0 | 0 | 0 | 0 | 0 | 0 | 0 | 0 |
| 10 | 2h | 0.5 | 1 | 1 | 1 | 0 | 0 | 0 | 0 | 0 | 0 | 0 | 0 | 0 | 0 | 0 |
| 11 | 2h | 0.8 | 1 | 1 | 1 | 1 | 1 | 1 | 1 | 0 | 0 | 0 | 0 | 0 | 0 | 0 |
| 12 | 2h | 0.9 | 1 | 1 | 1 | 1 | 1 | 1 | 1 | 1 | 1 | 1 | 1 | 1 | 1 | 0 |
| 13 | 2h | 1.0 | 1 | 1 | 1 | 1 | 1 | 1 | 1 | 1 | 1 | 1 | 1 | 1 | 0 | 0 |

CONDITION A         CONDITION B

# FIG. 30

Worst probability

# FIG. 31

RESULT OF TEST SAMPLE

SAMPLE NO. (S_No.) | ALL

CLOTTING TIME (sec) | ▶ | -

DISPLAY OF MAP | ALL

IDENTIFICATION ESTIMATION RESULT | ▶ | -

RESULT OF ESTIMATING ACTIVITY OF COAGULATION FACTOR | -

SUMMARY OF CMT SAMPLE IDENTIFICATION RESULT

130 ANALYSIS UNIT

120 CONTROL COMPUTER

124 COMMUNICATION INTERFACE

118c

## INTERNATIONAL SEARCH REPORT

| International application No. |
| --- |
| **PCT/JP2023/022245** |

| | |
| --- | --- |
| **A. CLASSIFICATION OF SUBJECT MATTER** | |

***G01N 33/86***(2006.01)i; ***G01N 33/48***(2006.01)i; ***G16H 10/40***(2018.01)i
FI:  G01N33/86; G01N33/48 Z; G16H10/40

According to International Patent Classification (IPC) or to both national classification and IPC

**B. FIELDS SEARCHED**

Minimum documentation searched (classification system followed by classification symbols)

G01N33/86; G01N33/48; G16H10/40

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
| --- | --- | --- |
| X | JP 2022-062969 A (SYSMEX CORP.) 21 April 2022 (2022-04-21)<br>  paragraphs [0010]-[0124], fig. 1-23 | 19-20 |
| X | WO 2020/256107 A1 (SEKISUI MEDICAL CO., LTD.) 24 December 2020 (2020-12-24)<br>  paragraphs [0013]-[0070], fig. 1, 2 | 19 |
| A | WO 2020/218425 A1 (SEKISUI MEDICAL CO., LTD.) 29 October 2020 (2020-10-29)<br>  entire text, all drawings | 1-29 |
| A | JP 2016-118442 A (NARA MEDICAL UNIVERSITY) 30 June 2016 (2016-06-30)<br>  entire text, all drawings | 1-29 |
| A | WO 2016/170944 A1 (NARA MEDICAL UNIVERSITY) 27 October 2016 (2016-10-27)<br>  entire text, all drawings | 1-29 |
| A | JP 2002-519635 A (CARDIOVASCULAR DIAGNOSTICS INC.) 02 July 2002 (2002-07-02)<br>  entire text, all drawings | 1-29 |

☐ Further documents are listed in the continuation of Box C.     ☑ See patent family annex.

| | |
| --- | --- |
| *    Special categories of cited documents:<br>"A"  document defining the general state of the art which is not considered to be of particular relevance<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |

| Date of the actual completion of the international search | Date of mailing of the international search report |
| --- | --- |
| **28 August 2023** | **05 September 2023** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT
### Information on patent family members

International application No.

**PCT/JP2023/022245**

| Patent document cited in search report | | | Publication date (day/month/year) | Patent family member(s) | | | Publication date (day/month/year) |
|---|---|---|---|---|---|---|---|
| JP | 2022-062969 | A | 21 April 2022 | US | 2022/0115091 | A1 | |
| | | | | paragraphs [0049]-[0178], fig. 1-23 | | | |
| | | | | EP | 3982110 | A1 | |
| | | | | CN | 114417690 | A | |
| WO | 2020/256107 | A1 | 24 December 2020 | US | 2022/0373565 | A1 | |
| | | | | paragraphs [0046]-[0130], fig. 1, 2 | | | |
| | | | | EP | 3988933 | A1 | |
| | | | | CN | 114008453 | A | |
| WO | 2020/218425 | A1 | 29 October 2020 | US | 2022/0326262 | A1 | |
| | | | | entire text, all drawings | | | |
| | | | | EP | 3961211 | A1 | |
| | | | | CN | 113785197 | A | |
| JP | 2016-118442 | A | 30 June 2016 | US | 2016/0178651 | A1 | |
| | | | | entire text, all drawings | | | |
| WO | 2016/170944 | A1 | 27 October 2016 | US | 2018/0045709 | A1 | |
| | | | | entire text, all drawings | | | |
| | | | | EP | 3287791 | A1 | |
| | | | | CN | 107533071 | A | |
| JP | 2002-519635 | A | 02 July 2002 | US | 6165795 | A | |
| | | | | entire text, all drawings | | | |
| | | | | WO | 1999/067630 | A1 | |
| | | | | EP | 1088223 | A1 | |
| | | | | KR | 10-2001-0071593 | A | |

Form PCT/ISA/210 (patent family annex) (January 2015)

**REFERENCES CITED IN THE DESCRIPTION**

**Patent documents cited in the description**

- JP 6994528 B
- WO 2020158948 A1
- JP 6811975 B
- US 6524861 B1

**Non-patent literature cited in the description**

- **D. SHIMOMURA et al.** *The First-Derivative Curve of the Coagulation Waveform Reveals the Cause of aPTT Prolongation, Clinical and Applied Thrombosis/Hemostasis*, 2020, vol. 26, 1-8